# EUROPEAN PATENT APPLICATION

(11) **EP 4 088 745 A1**
(43) Date of publication of application: **16.11.2022**
(21) Application number: 21173674.9
(22) Date of filing: 12.05.2021
(51) Int. Cl.: A61K 51/04, C07B 59/00, C07D 471/06

(54) **COMPOUND FOR THE DIAGNOSIS OF CANCER**

(71) Applicant: Eberhard Karls Universität Tübingen Medizinische Fakultät, 72074 Tübingen (DE)
(72) Inventor: Stotz, Sophie, 72074 Tübingen (DE); Bowden, Gregory David, 72074 Tübingen (DE); Maurer, Andreas, 72076 Tübingen (DE); Pichler, Bernd, 85298 Scheyern (DE)
(74) Representative: Witte, Weller & Partner Patentanwälte mbB

(57) **Abstract**

The present invention relates to a compound for the diagnosis of cancer, a compound for visualizing a PARP enzyme, to a method for the diagnosis of cancer in a living being, and to a method of visualizing a PARP enzyme in biological material.

## Description

The present invention relates to a compound for the diagnosis of cancer, a compound for visualizing a PARP enzyme, to a method for the diagnosis of cancer in a living being, and to a method of visualizing a PARP enzyme in biological material.

### FIELD OF THE INVENTION

The present invention relates to the field of molecular diagnostics, especially preclinical and clinical imaging and radiopharmacy.

### BACKGROUND OF THE INVENTION

The diagnosis of cancer is often made by the detection of so-called tumor markers in the examined individual. A tumor marker is a biomarker found in blood, urine, or body tissues that can be elevated by the presence of one or more types of cancer. There are many different tumor markers, each indicative of a particular disease process, and they are used in oncology to help detect the presence of cancer. An elevated level of a tumor marker can indicate cancer.

Poly (ADP-ribose) polymerase (PARP) is a family of proteins involved in a number of cellular processes such as DNA repair, genomic stability, and programmed cell death. PARP overexpression in several tumor entities has also made the enzyme a valuable bio- or tumor marker for optical and nuclear imaging techniques to detect malignant lesions and aid surgical excision of difficult-to-detect cancer entities.

Non-invasive imaging techniques such as positron emission tomography (PET) are used to track the fate of a radioactively labeled molecule *in vivo*; thus, aiding diagnosis and therapy surveillance. Quantification of PARP expression is thought to be predictive of tumor malignancy, as its overexpression is correlated with a poor clinical prognosis.

The PARP enzyme family consists of 17 members, of which PARP1 is the most abundant and best-characterized. It senses DNA single-strand breaks (SSBs) with its zinc finger domains and initiates their repair upon auto poly(ADP-ribosyl)ation (PARyla-tion), which recruits repair enzymes. The catalytic domains of PARP enzymes use nicotinamide adenine dinucleotide (NAD⁺) as the substrate for PARylation. PARP1, PARP2, and PARP5a and b (tankyrase 1 and 2) are capable of PAR chain formation while the remaining PARP enzymes can perform mono(ADP-ribosyl)ation (MARylation) or are enzymatically inactive.

PARP enzymes have also have become important targets for personalized cancer treatment, and this has spurred the development of several highly potent PARP inhibitors. PARP inhibitors induce synthetic lethality in malignant tumors that lack the homologous recombination (HR) DNA repair pathway.

PARP inhibitors structurally mimic NAD⁺, thus blocking the catalytic domain and escalating the SSBs to double-strand breaks (DSBs) that can only be repaired by alternative DNA repair mechanisms like HR. In addition to their catalytic inhibition, some PARP inhibitors are (to various extents) able to trap the PARP enzyme on the damaged DNA site, leading to replication fork collapse and ultimately cell death.

Within the last decade, numerous PARP inhibitors with various levels of cytotoxic efficacy have been approved by the U.S. Food and Drug Administration (FDA): olaparib (2014), rucaparib (2016), niraparib (2017), and talazoparib (2018). These inhibitors are mainly used in combination with DNA damaging agents like cisplatin in HR-deficient cancer entities or for maintenance therapy. Several ongoing studies are also exploring the potential of PARP inhibitors for monotherapy in HR-potent tumors. It is commonly agreed that substantial differences in the cytotoxic efficacies of the known PARP inhibitors are explained by the differences in their abilities to trap PARP1 on damaged DNA and not necessarily by their binding affinities alone.

Talazoparib is considered the most potent of the known PARP1 inhibitors and the best PARP trapping agent. The molecule possesses two distinct chiral centers and is present as two dominant enantiomers. The (8S, 9R)-diastereomer (talazoparib) is an excellent PARP inhibitor, while its enantiomer (LT-674) is less active by several orders of magnitude. It has been proposed that the bulky structure and stereochemistry of talazoparib contribute to its high potency, although the exact molecular interplay remains elusive.

PARP imaging has proven its exceptional clinical merit in several scenarios, specifically for the detection and delineation of oral, brain, pancreatic, and liver cancers, as well as target engagement imaging of different PARP inhibitors. Several PARP1-targeted imaging probes have been developed, with the most prominent among these being PARPi-FL, a fluorescently labeled olaparib analog; [¹⁸F]PARPi, PARPi-FL's radioactive sibling; [¹⁸F]olaparib, isotopically radiolabeled olaparib; and [¹⁸F]FTT, the first clinically applied PARP radiotracer.

However, the existing compounds for the diagnosis of cancer are characterized by a relatively low affinity to the targeted tumor marker or PARP, respectively, which requires a considerably high amount to be administered to the patient. This, in turn, results in a high radiation exposure of the patient. Further, the achieved contrast to healthy tissue is still not satisfactory.

It is, therefore, an object underlying the invention to provide a new compound for the diagnosis of cancer by means of which the disadvantages of the known compounds are overcome or at least reduced. In particular, such a compound is to be provided which can be used in an imaging method, such as PET.

This present invention satisfies this and other needs.

### SUMMARY OF THE INVENTION

This object is met by the provision of a compound of the formula 10 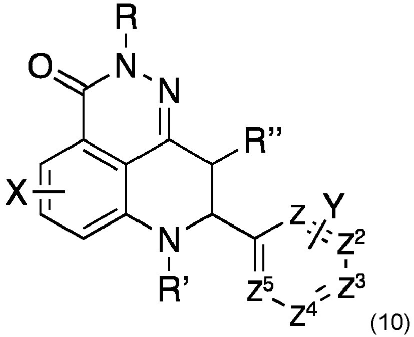 , wherein
R and R' are both H;
R" is a heteroaromatic or an aromatic residue, wherein R" has a molecular weight of 120 g/mol or less;
X and Y are independently selected from the group consisting of H, one or more leaving groups for [¹⁸F] fluorination, and a saturated or unsaturated alkyl residue of a molecular weight of 80 g/mol or less, provided that at least one leaving group for [¹⁸F] fluorination is present;
z and z²⁻⁵ are independently selected from the group consisting of C and N;
for use in the diagnosis of cancer, or, alternatively, for visualizing a poly [ADP-ribose] polymerase (PARP) enzyme.

The term 'heteroaromatic residue' pertains to residues that encompass a saturated cyclic compounds having 5 or 6 atoms that are selected from C, N, O, or S; preferably C or N. More preferred are saturated cyclic compounds having 4 C and 1 N atoms, 3 C and 2 N atoms, 2 C and 3 N atoms, 5 C and 1 N atoms, 4 C and 2 N atoms, or 3 C and 3 N atoms. Examples of preferred heteroaromatic residues involve me-1,2,4-triazol-5-yl, 4-me-1,2,4-triazol-3-yl, 4-pyridyl, 3-pyridyl, 2-pyridyl, 1-me-imidazol-2-yl, and 1-me-pyrazol-5-yl.

An 'aromatic residue' as used herein encompasses an unsaturated cyclic compound having 6 C-atoms. The 'aromatic residue' may exhibit one or more functional groups such as methyl or ethyl. A preferred aromatic residue is phenyl.

The molecular weight of the heteroaromatic residue or aromatic residue is 120 g/mol or less, preferably 110 g/mol or less, 100 g/mol or less, or 90 g/mol or less.

X and Y are selected from the group consisting of H, one or more leaving groups for [¹⁸F] fluorination, or a saturated or unsaturated alkyl residue of a molecular weight of 80 g/mol or less, such as 70 g/mol or less, 60 g/mol or less, 50 g/mol or less, 40 g/mol or less, or 30 g/mol or less, provided that at least one leaving group for [¹⁸F] fluorination is present.

The molecular sizes of the heteroaromatic residue, aromatic residue as well as the saturated or unsaturated alkyl residue ensures that that the [¹⁸F] talazoparib derivatives do not exhibit a sterical hindrance that negatively affects or even impedes the biological activity. In addition or alternatively, the retention time is not negatively affected, i.e. the retention may be similar to that of talazoparib.

Leaving groups for [¹⁸F] fluorination are well known in the art and include any leaving group susceptible to nucleophilic fluorination, such as Cl, Br, or I. Exemplary reactions involve the reactions of corresponding bromonium salts or iodonium salts with the cyclotron- produced [¹⁸F]-potassium fluoride in the presence of a phase-transfer reagent, such as Kryptofix-222.

It will be appreciated that other residues, such as nitro, trialkylamine, halogen, mesylate, tosylate, or triflate, may be susceptible to nucleophilic fluorination as well depending on the molecules structures, particularly the presence of electron withdrawing groups or atoms that are close to the leaving group for [¹⁸F] fluorination, i.e. that are located within the same ring structure as the leaving group for [¹⁸F] fluorination.

It will be understood that not all leaving groups for ¹⁸F fluorination are substituted by ¹⁸F in course of the present method. Rather one or more leaving groups, such as one, two, or three leaving groups, may remain in the compound of formula 10a and/or 10b provided that at least one leaving groups for ¹⁸F fluorination is in fact converted to ¹⁸F.

It has been found in course of studies leading to the present invention that the concepts of nucleophilic fluorination are stretched to its limits in context with the present compounds. The reason essentially resides in the presence of nitrogen based heterocycles, which are believed to complex the copper mediator and forming unreactive copper species that inhibit radiosynthesis performance. It is therefore assumed that particularly suitable leaving groups [¹⁸F] fluorination include boronic acids, boronic acid esters, iodonium salts, iodonium ylids, trialkylstannanes, a nitro group, trialkyl ammonium salts, and sulfonate esters, such as tosylate (OTs), mesylate (Oms), nosylate (ONs), brosylate (OBs), or trifluoromethanesulfonate (OTf).

It is preferred that the compound of formula 10 has a single ¹⁸F radiolabel, preferrably ¹⁸F at the position C5 or z³, more preferably ¹⁸F at the position z³. Radiolabeling at these positions is expected to have no disadvantageous effects on the retention time and biological activity of the molecules.

The inventors have realized that the new compound is suitable for the diagnosis of cancer due to its binding affinity to poly [ADP-ribose] polymerase (PARP) enzymes, which is significantly higher than the affinity of known PARP binding or inhibiting compounds such as olaparib, rucaparib, or niraparib. E.g. the compound according to the invention has an effectivity which is up to 100 fold higher than such of the aforementioned compounds. For this reason, the compound according to the invention provides better contrast to healthy tissue with lower radiation exposure. Furthermore, in contrast to the afore-mentioned known PARP inhibitors, especially olaparib, the compound according to the invention not only binds to the PARP 1 and PARP 2 isoforms but also to the PARP isoforms of tankyrase 1 and tankyrase 2 (PARP5a/b). Thus, the compound according to the invention also offers the possibility to visualize this enzyme class and to gain new insights. By blocking PARP1/2 binding in advance with PARP1/2 specific olaparib or comparable PARP1/2 inhibitors, the compound according to the invention binds only to tankyrase enzymes, allowing them to be detected *in vivo.*

The provision of the compound according to the invention was made possible by the development of a novel method for [¹⁸F]-labeling of PARP inhibitors, which is further described below and which is the subject of a parallel patent application.

In an embodiment of the invention said cancer is selected from the group consisting of: oral cancer, brain cancer, pancreatic cancer, liver cancer, breast cancer and ovarian cancer.

By this measure the compound according to the invention makes use in an advantageous manner of the findings already observed for other PARP inhibitors, such as olaparib. The latter inhibitor has already been successfully used in the diagnosis of such tumors. However, due to its higher affinity to PARP enzymes the compound according to the inventions provides better contrast to healthy tissue with lower radiation exposure.

In another embodiment of the invention said PARP enzyme is tankyrase, preferably tankyrase 1 and/ or tankyrase 2.

This measure has the advantage that a radio-labelled compound is provided which, other than olaparib or comparable PARP1/2 Inhibitors, also allows the visualization of tankyrase. Due to its affinity to both enzyme classes (PARP1/2 and tankyrase), the compound according to the invention offers the ability to visualize tankyrases significantly faster than tankyrase-only radiotracers, which have not yet been developed.

In another embodiment of the invention the compound is [¹⁸F] talazoparib.

By this measure the compound according to the invention is configured as the eponymous PARP inhibitor initially developed by Pfizer for the treatment of advanced breast cancer with germline BRCA mutations, however, for the very first time provided as a radiotracer. 'Talazoparib', sold under the brand name Talzenna, was approved in October 2018, in the United States and June 2019, in the European Union for germline BRCA-mutated, HER2-negative locally advanced or metastatic breast cancer. Talazoparib acts as an inhibitor of poly ADP ribose polymerase (PARP) which aids in single strand DNA repair. Cells that have BRCA1/2 mutations are susceptible to the cytotoxic effects of PARP inhibitors because of an accumulation of DNA damage. Talazoparib is theorized to have a higher potency than olaparib due to the additional mechanism of action called PARP trapping. PARP trapping is the mechanism of action where the PARP molecule is trapped on the DNA, which interferes with the cells ability to replicate. Talazoparib is found to be -100 fold more efficient in PARP trapping than olaparib.

The term 'talazoparib' designates the CAS Name of the compound (8*S*,9*R*)-5-Fluoro-8-(4-fluorophenyl)-2,7,8,9-tetrahydro-9-(1-methyl-1*H*-1,2,4-triazol-5-yl)-3*H*-pyrido[4,3,2-de]phthalazin-3-one. Talazoparib is the biologically active enantiomer and is also known as BMN 673. The biologically inactive (8R, 9S) enantiomer of talazoparib is also designated as "LT-674".

The expression '[¹⁸F] talazoparib' as used herein refers to talazoparib with one or both fluorine atoms subsituted by ¹⁸F, i.e. the compounds (8*S*,9*R*)-5-¹⁸Fluoro-8-(4-fluorophenyl)-2,7,8,9-tetrahydro-9-(1-methyl-1*H*-1,2,4-triazo1-5-yl)-3*H*-pyrido[4,3,2-*de*]phthalazin-3-one, (8*S*,9*R*)-5-Fluoro-8-(4-¹⁸fluorophenyl)-2,7,8,9-tetrahydro-9-(1-methyl-1*H*-1,2,4-triazol-5-yl)-3*H*-pyrido[4,3,2-de]phthalazin-3-one, and (8*S*,9*R*)-5-¹⁸Fluoro-8-(4-¹⁸fluorophenyl)-2,7,8,9-tetrahydro-9-(1-methyl-1*H*-1,2,4-triazol-5-yl)-3*H*-pyrido[4,3,2-de]phthalazin-3-one. The compound of formula 10 includes the above mentioned compounds and the remaining compounds of formula 10 also referred to '[¹⁸F] talazoparib derivatives', which are also included in the invention.

In an embodiment of the invention said diagnosis or said visualizing is made by an imaging method.

This measure has the advantage that the compound according to the invention is configured for a use in routine diagnostic practise.

In a further embodiment of the invention said imaging method is positron emission tomography (PET).

By this measure the compound of the invention is adapted to the method of choice when using [¹⁸F]. PET is a common imaging technique, a medical scintillography technique used in nuclear medicine.

The invention also relates to a pharmaceutical composition, preferably a radiopharmaceutical, comprising the compound according to the invention and a pharmaceutically acceptable carrier.

A 'pharmaceutical composition' is a composition suitable for an administration to an animal and/or human being in a medical setting. Preferably, a pharmaceutical composition is sterile and produced according to GMP guidelines.

In an embodiment the compound according to the invention can be used as the only active agent of the pharmaceutical composition, i.e. without any additional further active agent.

Pharmaceutically acceptable carriers are well known in the art and include, for example, aqueous solutions such as water or physiologically buffered saline or other solvents or vehicles such as glycols, glycerol, oils such as olive oil or injectable organic esters. In preferred embodiments, when such pharmaceutical compositions are for human administration, e.g., for parenteral administration, the aqueous solution is pyrogen-free, or substantially pyrogen-free. The excipients can be chosen, for example, to effect delayed release of an agent or to selectively target one or more cells, tissues or organs. The pharmaceutical composition can be in dosage unit form such as tablet, capsule (including sprinkle capsule and gelatin capsule), granule, powder, syrup, suppository, injection or the like. The composition can also be present in a transdermal delivery system, e.g., a skin patch. The composition can also be present in a solution suitable for topical administration, such as an eye drop. Alternatively, the pharmaceutical composition can be present in form of an aerosol administrable by inhalation.

The features, embodiments and advantages of the compound according to the invention apply to the pharmaceutical composition in a corresponding manner, even if not specifically indicated.

The invention also relates to a method for the diagnosis of cancer in a living being, comprising the step of introducing the compound or the pharmaceutical composition according to the invention into said living being, and a step of visualizing said cancer by an imaging method, preferably by positron emission tomography (PET).

A 'living being' according to the invention, includes any being, especially a mammal, including a human being.

The invention further relates to a method of visualizing a poly [ADP-ribose] polymerase (PARP) enzyme in biological material, preferably tankyrase 1/2, comprising the step of introducing the compound according to the invention into said biological material, and a step of visualizing said PARP enzyme by an imaging method, preferably by positron emission tomography (PET).

'Biological material' refers to any biological material suspected of comprising PARP enzymes, including biological cells, tissue, organs and living beings.

The features, embodiments and advantages of the compound according to the invention apply to the methods according to the invention in a corresponding manner, even if not specifically indicated.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

### BRIEF DESCRIPTION OF THE FIGURES

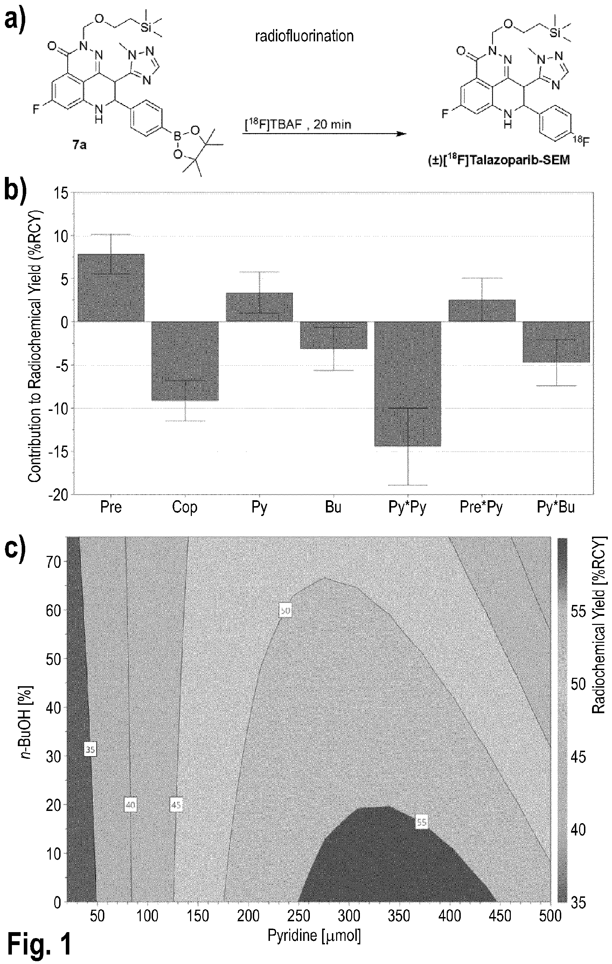

- Fig. 1:: Fig. 1A shows a D-Optimal DoE study that was used to optimize the radiolabeling of the compound 7a. Fig. 1B shows the scaled and centered regression coefficient calculated from the results of D-optimal response surface modeling DoE of the radiosynthesis of [¹⁸F] talazoparib. Large bars represent factors with a large contribution to the response (% radiochemical yield (RCY)). A positive number denotes a positive influence on the response. A negative number indicates a diminishing effect on the response. If a factor's regression coefficient is smaller than the associated error bars, it is probable (at the 95% confidence interval) then that factor is not significant. N=23 (sample size); R²=0.946 (good-ness of model fit); RSD=4.412 (residual standard deviation); D=15 (degrees of freedom); Q²=0.855 (goodness of model prediction); Confidence=0.95. Fig. 1C shows a section of the response surface calculated from the regression model (variables not shown: Cop = 5 µmol, Pre = 30 µmol). All data calculated in MODDE Go 12 software (Sartorius).
- Fig. 2:: shows another representation of the scaled and centered regression coefficient calculated from the results of D-optimal response surface modelling DoE of the radiosynthesis of [¹⁸F] talazoparib according to Fig. 1B.

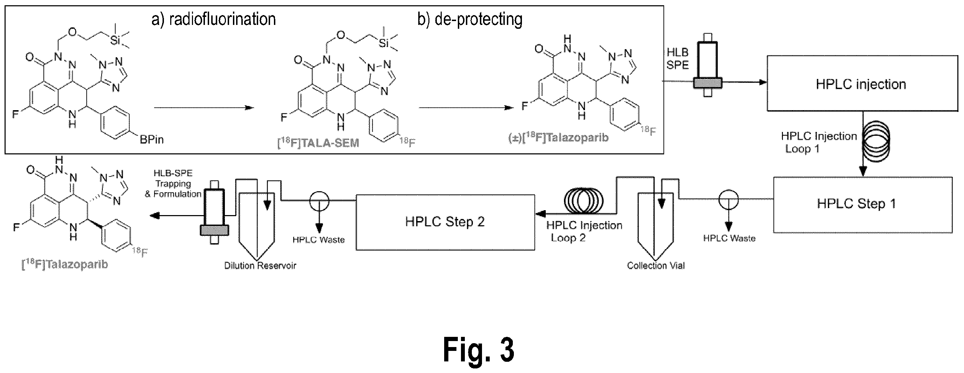

- Fig. 3:: shows the general automated procedure for the radiosynthesis, HLB-SPE clean-up, 2D HPLC purification and chiral resolution, and product concentration and formulation of [¹⁸F] talazoparib. The rectangular box indicates the radiosynthesis step including a) radiofluorination with [¹⁸F]TBAF, Cu(OTf)₂, Py, DMA, n-BuOH, 120°C for 20 min. and b) de-protecting with HCI 6M, 120°C for 10 min. followed by NaOH 6M, in HPLC buffer. The formulation for HPLC injection includes MeCN : water (0.1% TFA) (1:4). HPLC Step 1 includes C18 HPLC MeCN : water (0.1% TFA) (28:72). HPLC Step 2 includes Chiralpak IB-N5 MeCN : water (0.1% TFA) (40:60). The volume of the HPLC injection loops 1 and 2 as well as the collection vial is 5 ml, respectively. The volume of the dilution reservoir is 60 ml.
- Fig. 4:: shows summary statistics for D-Optimal DoE for CMRF of [¹⁸F] talazoparib. R2 represents the goodness of regression model fit. Q2 represents the goodness of model prediction. "Reproducibility" is calculated from the standard deviation in replicate (centerpoint) experiment results. These statistics represent a valid and predictive regression model.

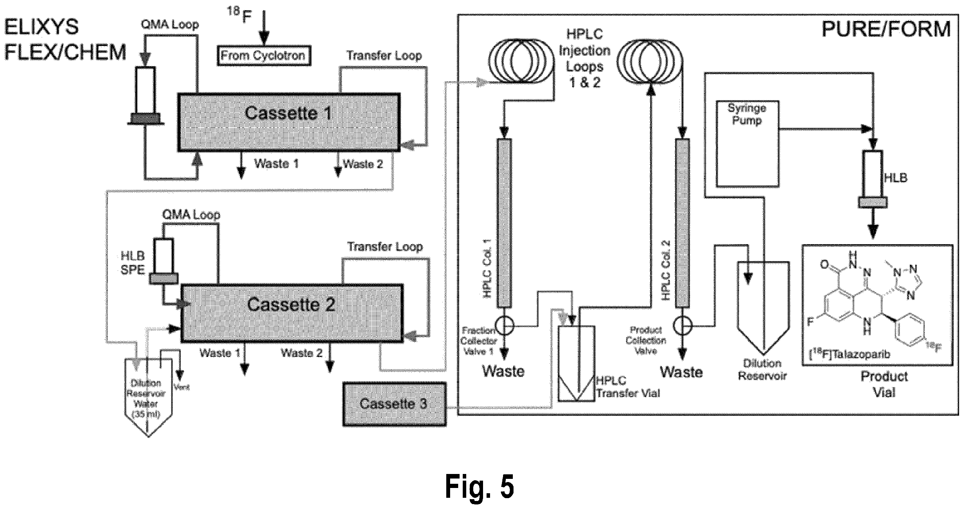

- Fig. 5:: shows the connection schematic for the fully automated [¹⁸F] talazoparib radiosynthesis using an Elixys FLEX/CHEM radiosynthesizer coupled to a PURE/FORM formulation and purification module. The cassette activity-in line is labelled ¹⁸F from cyclotron. Quaternary methylammonium (QMA) loops are cassette cartridge loop one and two, respectively (cassette one = QMA; cassette two = HLB). The transfer loops of Cassette 1 and Cassette 2 are cassette cartridge loop two, respectively (no cartridge). The arrow connecting Cassette 1 and the dilution reservoir is the cassette activity-out line.
- Fig. 6:: shows the set-up of the FX N Pro.
- Fig. 7:: A) Uptake of [¹⁸F] talazoparib (left) and the inactive enantiomer [¹⁸F]LT-674 (right) in HCC1937 cells, blocked with olaparib or talazoparib. B) Serum stability analysis in mouse (left) and human serum (right).
- Fig. 8:: shows the analytical Chiral HPLC (Chiralpak IB-N5, 5 µm, 150 mm x 4.6 mm) with MeCN: H₂O + TFA (0.1%); 40:60. Fig 8 a) shows a racemic mixture (non-radioactive standard). Fig 8 b) shows talazoparib (non-radioactive std.), and Fig 8 c) shows the inactive enantiomer LT-674 (non-radioactive std.).
- Fig. 9:: shows a semipreparative Radio-HPLC (IB-N5, 5 µm, 250 mm x 10 mm) MeCN: H₂O + TFA (0.1%); 40:60. The Radio-HPLC with the system of ELIXYS. Peak cuts are affected by the fraction collector.
- Fig. 10:: shows the analytical Chiral Radio-HPLC (IB-N5, 5 µm, 150 mm x 4.6 mm) with MeCN: H₂O + TFA (0.1%); 40:60. A and B show [¹⁸F] talazoparib (active enantiomer) with a retention time of about 5.8 min, and B and [¹⁸F]LT-674 (inactive enantiomer) with a retention time of about 7.375 min.
- Fig. 11:: Uptake of [¹⁸F]talazoparib in HCC1937 cells, blocked with different concentrations of olaparib or talazoparib.
- Fig. 12:: A) Representative PET images of one mouse 1 h p.i. (left, last 10 minutes of a 1 h dynamic scan) and 2 h p.i. (right, 10 minute static scan) with the corresponding MR image. Respective enlarged xenograft is displayed below. B) Biodistibution of [¹⁸F]talazoparib in various organs (n = 5). C) TMR (tumor-to-muscle ratio), TBR (tumor-to-brain ratio) and LKR (liver-to-kidney ratio) of mice injected with [¹⁸F]talazoparib 2.5 h p.i.
- Fig. 13:: Affinities of talazoparib and olaparib for Tankyrase 1 and 2 (TNKS1/2). A Affinity of talazoparib for TNKS1 and B TNKS2 C Affinity of olaparib for TNKS1 and D TNKS2 in comparison to the TNKS inhibitor XAV939.
- Fig. 14:: TACs displaying the uptake in the different organs dynamically over a time course of 1 h after injection of [¹⁸F]talazoparib.
- Fig. 15:: Representative Immunofluorescence Images of HCC1937 xenograft tissue. PARP1 is displayed in red and nuclei are stained in green. Scale bars represent 100 µm (left image), 20 µm (middle image) and 10 µm (right image).

### EXAMPLES

### 1. Experimental

### General

All chemicals, reagents, catalysts, and solvents were purchased from either *Sigma Aldrich* (St. Louis, Missouri, USA), *Merck* (Darmstadt, Germany), *abcr GmbH* (Karlsruhe, Germany), *Karl Roth* (Karlsruhe, Germany), and were used without any additional purification unless otherwise stated. QMA, SPE, and SEP-PAK cartridges were obtained from *Waters* (Milford, Massachusetts, USA) unless otherwise stated. Reactions were monitored using thin-layer chromatography (TLC) on 0.20 mm Polygram SIL G/UV₂₅₄ (silica gel 60) TLC plates and were developed with an appropriate running buffer/solvent mixture. Spots were visualized with UV light (254 or 366 nm). Preparative flash chromatography was performed using pre-packed silica gel columns (SNAP KP-Sil or SNAP Ultra (25 µm HP-Sphere), 10 g, 25 g, 50 g, or 100 g, (*Biotage,* Uppsala, Sweden)) on an automated chromatography system (Isolera 4, *Biotage*) which featured a UV detector and fraction collector. Unless otherwise stated, all columns were dry loaded by absorption onto either silica gel or diatomaceous earth packing material (Isolute, *Biotage).* ¹H and ¹³C NMR spectra were obtained at 300 K using an Avance III AV 600 (¹H: 600.13 MHz and ¹³C: 150.61 MHz) spectrometer (*Bruker,* Billerica, Massachusetts, USA). All chemical shifts (δ) are reported in ppm, and all *J* values are reported in Hz. The following abbreviations are used to describe multiplicities: s (singlet), d (doublet), t (triplet), q (quartet), m (multiplet) brs (broad singlet). All compounds were dissolved in chloroform (CDCl₃) unless otherwise stated. All chemical shifts were referenced to residual chloroform (δ_{H} = 7.24 and δ_{C} = 77.00) or DMSO (δ_{H} = 2.50 and δ_{C} = 39.52). Analytical HPLC-MS data was collected using a 1200 series HPLC machine coupled to quadrupole 6120 series MS detector in ESI mode (*Agilent,* Santa Clara, California, USA) under the following conditions: Column: Luna 5µm C18 (2) 100 Å, 50 x 2 mm; Solvent A: H₂O + Formic acid (0.1%); Solvent B: acetonitrile; Gradient: 0-7.60 min (0% - 100% B), 7.60 - 7.80 min (100% B), 7.80 - 8.30 min (100% - 0% B), 8.30 - 12.0 min (0% B).

### Chemical Synthesis

*1-Methyl-1H-1,2,4-triazole-5-carbaldehyde* (1). 1-methyl-1H-1,2,4-triazole (10 g, 120 mmol) was dissolved in THF (60 ml) in a dry argon purged two-neck reaction flask fitted with a rubber septum, and the resulting solution was then cooled to 0 °C. A solution of 2M isopropylmagnesium chloride (66 ml, 132 mmol) was then added dropwise through the septum via a syringe over 15 min. The reaction mixture was then allowed to warm slowly to room temperature and stir for 1.5 hours. The reaction vessel was again cooled to 0 °C, after which N,N-dimethyl formamide (DMF) (14 ml, 180.5 mmol) was added dropwise via syringe. The reaction mixture was once more allowed to slowly warm to room temperature and stir overnight. The next morning, the reaction was quenched by the slow addition of HCl (2M) until pH 2, and the resulting mixture was diluted with DCM (100 ml). The phases were separated with a separating funnel, and the aqueous phase was neutralized with saturated aqueous sodium bicarbonate (NaHCO₃) and extracted with DCM (2 x 80 ml). The organic fractions were combined, washed once with brine, dried with magnesium sulfate (MgSO4), and concentrated under reduced pressure to remove the DCM. The product 1 is a volatile oil (≈60 °C) and was thus not purified further. The compound was used, in excess, directly in the next step without further analysis, assuming a synthesis yield of 75%.

*6-Fluoro-3-((1-methyl-1H-1,2,4-triazol-5-yl)methylene)-4-nitroisobenzofuran-1(3H)-one (2).* Commercially available 6-fluoro-4-nitroisobenzofuran-1(3*H*)-one (9.8 g, 50 mmol), a solution of 1 in THF (ca. 100 mmol, assuming 75% conversion in the previous step), and triethylamine (21 ml, 150 mmol) were dissolved in dry THF (150 ml) in an argon-filled two-neck reaction flask fitted with a reflux condenser. Acetic anhydride (35 ml) was then added dropwise to the reaction mixture over 3 min, and the resulting mixture was then heated to reflux for 1 hour. The mixture was then removed from the heat and concentrated under reduced pressure to a volume of approximately (10 ml) until a green/yellow precipitate formed. The resulting slurry was then cooled in a freezer to -5 °C, and the solid was collected through vacuum filtration. The resulting cake was washed with cold ethyl acetate, and the residue was then dried under high vacuum for 4 hours to afford 2 as a grey-green solid (7.34 g, 50 %). The analytical data agree with the literature. 1H NMR (600 MHz, DMSO) δ 7.74 (dd, J = 8.7, 2.3 Hz, 1H), 7.56 (dd, J = 6.4, 2.4 Hz, 1H), 7.25 (d, J = 0.6 Hz, 1H), 6.32 (s, 1H), 3.09 (s, 3H).

*Methyl 5-fluoro-2-(2-(1-methyl-1H-1,2,4-triazol-5-yl)acetyl)-3-nitrobenzoate (**3**).* Compound 2 (7.34 g, 25.2 mmol) was suspended in methanol (204 ml), and acetic acid (0.5 ml) was added to the resulting solution. The mixture was then warmed to 50 °C until HPLC-MS confirmed complete consumption of the starting material (4 - 12 hours). The solvents were then removed under high vacuum to afford 3 as a yellow solid in nearly a quantitative yield (8.1 g, 99%). The product was used directly in the next step without further purification. The analytical data agree with the literature. 1H NMR (600 MHz, DMSO) δ 8.52 (dd, J = 8.2, 2.6 Hz, 1H), 8.26 (dd, J = 8.3, 2.6 Hz, 1H), 7.85 (s, 1H), 4.59 (s, 2H), 3.91 (s, 3H), 3.88 (s, 3H). 13C NMR (151 MHz, DMSO) δ 195.48, 163.48, 161.21 (d, J = 252.6 Hz), 150.18, 148.75, 147.10 (d, J = 8.8 Hz), 133.58, 131.24 (d, J = 7.7 Hz), 123.63, 117.09 (d, J = 26.9 Hz), 53.71, 40.55, 35.34.

*8-(4-Bromophenyl)-5-fluoro-9-(1-methyl-1H-1,2,4-triazol-5-yl)-2,7,8,9-tetrahydro-3H-pyrido[4,3,2-de]phthalazin-3-one **(5).*** Compound **3** (8.1 g, 25.2 mmol) and 4-bromobenzaldehyde (8.9 g, 50.5 mmol) were suspended in THF (50 ml) and MeOH (10 ml). To the resulting mixture was added titanium (III) chloride solution (20% wt solution in HCI (2M), 130 ml, 6 Eq.) in dropwise fashion over 30 min at room temperature. The reaction temperature was maintained between 30 and 50 °C for 2 hours, after which it was quenched by the slow addition of water (260 ml). The reaction mixture was then poured into a separating funnel and extracted with ethyl acetate (4 x 140 ml). The organic fractions were pooled and washed with NaHCO₃ (3 x 60 ml) and NaHSO₃ (3 x 100 ml), dried with sodium sulfate (NaSO₄), and concentrated under reduced pressure to afford a think yellow syrup, which was carefully washed with aliquots of diethyl ether (3 x 10 ml). The resulting yellow syrup was then dried under high vacuum to afford the crude intermediate **4** as a yellow amorphous solid (11.3 g, 98%) that was used in the next step without any further purification.

Intermediate **4** (11.3 g, 24.6 mmol) was dissolved in methanol (30 ml) at room temperature, and to the resulting solution was added hydrazine monohydrate (7.7 ml). The reaction mixture was then left to stir overnight at room temperature. The next morning the resulting white precipitate was collected via vacuum filtration to afford Compound **5** as an off-white solid (4.9g, 45% over two steps). 1H NMR (600 MHz, DMSO) δ = 12.35 (s, 1H), 8.04 (s, 0H), 7.80 (s, 1H), 7.72 (s, 1H), 7.53 (d, J=8.2, 2H), 7.41 (d, J=8.1, 2H), 7.07 (dd, J=8.9, 2.4, 1H), 6.91 (dd, J=11.1, 2.5, 1H), 5.04 - 4.96 (m, 2H), 4.02 (s, 1H), 3.68 (s, 3H).

*8-(4-Bromophenyl)-5-fluoro-9-(1-methyl-1H-1,2,4-triazol-5-yl)-2-((2-(trimethylsilyl)ethoxy)methyl)-2,7,8,9-tetrahydro-3H-pyrido[4,3,2-de]phthalazin-3-one **(6a)** and 8-(4-Bromophenyl)-5-fluoro-9-(1-methyl-1H-1,2,4-triazol-5-yl)-2,7-bis((2-(trimethylsilyl)ethoxy)methyl)-2,7,8,9-tetrahydro-3H-pyrido[4,3,2-de]phthalazin-3-one **(6b).*** Sodium hydride (60% in mineral oil, 2.7 mg, 6.8 mmol) was suspended in DMF (20 ml) in a dry, argon-filled flask, and the resulting solution was then cooled to -15- -18 °C using a bath containing a mixture of acetone and ice. Compound **5** (2 g, 4.5 mmol) was added to the NaH suspension in small portions, and DMF (1 ml) was used to wash any compound off the vessel walls. The deep red/purple solution was allowed to stir for 15 minutes, whereupon a solution of (trimethylsilyl)ethoxymethyl chloride (1.13 g, 6.8 mmol) in DMF (4 ml) was added dropwise through a rubber septum over 5 minutes. Throughout the addition, the solution turned clear and light orange and was allowed to stir for a further 20 min before being quenched with sat. Aq. NH₄Cl (20 ml). The reaction mixture was diluted with water (250 ml) and transferred to a separating funnel, where it was extracted with ethyl acetate (3 x 50 ml). The organic fractions were pooled, dried with MgSO₄ and evaporated to dryness. The crude product residue was then purified using flash chromatography (25-75% EtOAc in hexanes) to afford compounds **6a** and **6b** as a white crystalline solid and thick colorless oil (**6a:** 1.447 g, 56 %; **6b:** not determined). Despite the presence of several minor impurities both compounds were used in the next step without further purification.

*5-fluoro-9-(1-methyl-1H-1,2,4-triazol-5-yl)-8-(4-(4,4,5,5-tetramethyl-1,3,2-dioxaborolan-2-yl)phenyl)-2-((2-(trimethylsilyl)ethoxy)methyl)-2,7,8,9-tetrahydro-3H-pyrido[4,3,2-de]phthalazin-3-one **(7a).*** Compound ***6a*** (1.448 g, 2.534 mmol), bis(pinacolato)diboron (1.415 g, 5.574 mmol), and potassium acetate (746 mg, 7.602 mmol) were suspended in DMF (22 ml) in a dry argon filled reaction flask fitted with a reflux condenser. [1,1'-Bis(diphenylphosphino)ferrocene]dichloropalladium(II) (Pd(dppf)Cl₂, 371 mg, 0.507 mmol, 20 mol%) was added to reaction mixture and DMF (3 ml) was used to wash the catalyst of the vessel walls. The reaction flask was purged with argon, and the reaction was heated to 90 °C for 4 hours until HPLC-MS showed conversion of the starting material into the desired product. The reaction mixture was diluted with ethyl acetate (80 ml) and passed through a tightly packed plug of Celite^{®} under vacuum. The filtrate was collected and transferred to a separating funnel where it was washed with water (200 ml). The aqueous layer was further extracted with ethyl acetate (3 x 80 ml), after which the organic fractions were pooled, washed with brine, dried with MgSO4, and concentrated under reduced pressure to afford the desired product racemate (7a) as an off-white solid (1.391 g, 89%).

For radiochemical experiments, the product (515 mg) was further purified by recrystallization. The product was dissolved completely in a hot mixture of acetone and acetonitrile (1:1), and deionized water was then added dropwise until the solution became turbid. The solution was again lightly heated until the solution was nearly transparent, and it was then allowed to slowly cool to the ambient temperature, after which the recrystallization vessel was placed in a refrigerator at 4 °C and allowed to sit overnight. The next day the pure product was collected via vacuum filtration to afford a pure racemic mixture of **7a** as a white crystalline solid (361 mg, 70% recovery). H1 and C13, HPLC: HRMS 1H NMR (600 MHz, CDCl3) δ 7.85 (s, 1H), 7.73 (d, J = 7.8 Hz, 2H), 7.41 (dd, J = 9.7, 2.3 Hz, 1H), 7.36 (d, J = 7.8 Hz, 2H), 6.75 (dd, J = 9.7, 2.3 Hz, 1H), 5.43 (d, J = 9.9 Hz, 1H), 5.26 - 5.18 (m, 2H), 5.02 (s, 1H), 4.52 (d, J = 11.0 Hz, 1H), 3.57 (s, 3H), 2.44 - 1.60 (m, 4H), 1.33 (s, 12H), 0.92 - 0.84 (m, 2H), 0.0 (s, 9H).

The present inventors adapted the synthesis of the precursor from the synthetic route employed by Wang *et al.* for the synthesis of talazoparib (Wang, B.; Chu, D.; Feng, Y.; Shen, Y.; Aoyagi-Scharber, M.; Post, L. E., Discovery and Characterization of (8S,9R)-5-Fluoro-8-(4-fluorophenyl)-9-(1-methyl-1H-1,2,4-triazol-5-yl)-2,7,8,9-te trahydro-3H-pyrido[4,3,2-de]phthalazin-3-one (BMN 673, talazoparib), a Novel, Highly Potent, and Orally Efficacious Poly(ADP-ribose) Polymerase-1/2 Inhibitor, as an Anticancer Agent. J Med Chem 2016, 59 (1), 335-57). It will be appreciated, however, that also other synthetic routes may be used as starting points for the provision of the precursor of formula 5c.

### Precursor synthesis

The precursor of the formula 5c was synthesized as mentioned hereinafter. As may be derived from scheme 1, 1-methyl-1H-1,2,4-triazole was formylated to 1 via the published procedure (Albrecht, Brian K.; Bauer, David; Bellon, Steven; Bode, Christiane M.; Booker, Shon; Boezio, Alessandro; Choquette, Deborah; D'Amico, Derin; Harmange, Jean-Christophe; Hirai, Satoko; Hungate, Randall W.; Kim, Tae-Seong; Lewis, Richard T.; Liu, Longbin; Lohman, Julia; Norman, Mark H.; Potashman, Michelle; Siegmund, Aaron C.; Springer, Stephanie K.; Stec, Markian; Xi, Ning; Yang, Kevin, (2009) FUSED HETEROCYCLIC DERIVATIVES AND METHODS OF USE; WO 2009/091374). **1** was then condensed with commercially available 6-fluoro-4-nitroisobenzofuran-1(3H)-one in THF in the presence of NEt₃ and Ac₂O to afford **2** in a 53% yield. The lactone **2** was opened to give the keto-ester derivative **3** by warming **2** in methanol with a catalytic amount of acetic acid. In the original synthesis, **3** was then reacted with 4-fluorobenzaldehyde in THF and methanol via reductive cyclization driven by TiCl₃ to afford **4b,** which was cyclized with hydrazine monohydrate to afford (±) talazoparib (**5b**), which was to be used as reference compound. Reacting **3** with 4-bromobenzaldehyde via the same two-step procedure afforded the derivatizable bromide **5a.**

A protecting group strategy using the trimethylsilylethoxymethyl ether (SEM) protecting group was employed to the compound **5a,** which was protected with trimethylsilylethoxymethyl chloride (SEMCI) using sodium hydride in DMF at 15 °C. These conditions afforded both the mono- (**6a**) and di-SEM (**6b**) protected bromide derivatives. It is clear, however, that the protecting group strategy is not limited to the disclosed compounds.

Compounds **6a** and **6b** were then further derivatized to yield several possible precursors for radiolabeling (Scheme 2). The protection of **5a** favored the formation **6a** over **6b** (ca. 3:1), even under conditions designed to drive the formation of the di-SEM protected derivative. Compounds **6a** and **6b** were converted to their corresponding boronic acid pinacol esters under Miyaura borylation conditions in DMF with KOAc, bis(pinacolato)diboron, and Pd(dppf)Cl₂ as the catalyst (Scheme 2). This afforded the compounds **7a** and **7b** in good to excellent yields. **7a** was purified through recrystallization a shelf-stable and convenient-to-handle white crystalline solid. **7b** however, was purified through chromatography and was much harder to obtain in the high purities required for reliable radiochemistry. Despite being of high chromatographic purity (HPLC-MS), samples of **7b** displayed a complex NMR spectrum, indicative of the presence of multiple conformation isomers. CMRF chemistry has also been well established with aryl stannanes precursors and the present inventors have investigated this variation of the CMRF reaction extensively in our previous work. Thus, the analogous stannylation of 6a was carried with bis tributyltin to afford the stannyl precursor **7c** as an amorphous glass.

Optimal chiral HPLC conditions were screened using racemic (±)talazoparib in combination with commercially obtained authentic samples of talazoparib and LT-674. CHIRALPAK IA-U, IB-U, IC-U and IG-U UHPLC columns were tested for their abilities to resolve the two enantiomers under reverse-phase conditions (Water (0.1% TFA):Acetonitrile). CHIRALPAK IB packing material was found to provide a good enantiomeric resolution at relatively low retention time. CHIRALPAK IB-N5 (5 µm) columns under reverse-phase conditions would thus be used for both semipreparative enantiomeric resolution of [¹⁸F] talazoparib and [¹⁸F]LT-674 and for chiral quality control (QC) analysis.

### Automated Radiochemistry and DoE Studies

With the precursors **7a-c** in hand, the inventors set out to evaluate which precursor would provide the best radiosynthesis performance, while still being relatively easy to produce, shelf-stable, and convenient to work with. A series of pilot experiments, carried out using each precursor under a small set of arbitrarily chosen unoptimized reaction conditions, determined that compounds **7b** and **7c** displayed only marginally better reaction performance (data not shown) than **7a** (8-15% RCY using unoptimized reaction conditions). As **7a** proved easier to synthesize, purify, and characterize, as well as being shelf-stable, it was chosen as the precursor for further development and optimization.

All radiochemical reactions and experiments were carried out behind appropriate shielding in accordance with the rules and guidelines laid out in the German Strahlenschutzverordnung (StrlSchV).

The inventors have previously explored the use of DoE to solve complex radiochemical optimization problems and have developed a ¹⁸F processing method and workflow that is compatible with small scale DoE radiochemical experiments shown in DoE Studies hereinafter. The inventors applied this workflow to a computer-generated D-optimal DoE study designed investigate the effects of precursor load (*Pre,* 5-30 µmol), the copper-mediator load (*Cop*, 5-40 µmol), the pyridine load (*Py,* 20-500 µmol), and the % of n-BuOH co-solvent (*Bu*, 0-75%) in DMA, on reaction performance (Figs. 1A and 1B). The study consisted of 24 experiments (including centerpoints) and was conducted over 4 days (5 runs/day) using [¹⁸F] fluoride from cyclotron target washes (Table S1). Each run was carried out for 20 minutes at 120 °C before evaluation by radioTLC. Analysis of the data revealed the presence of one outlier (sup table. exp 21) which was excluded from the final regression model. The remaining data (n = 23) was used to fit a regression model with R² = 0.946 (goodness of model fit) and Q² = 0.855 (goodness of model prediction) suggesting the model to be valid and predictive.

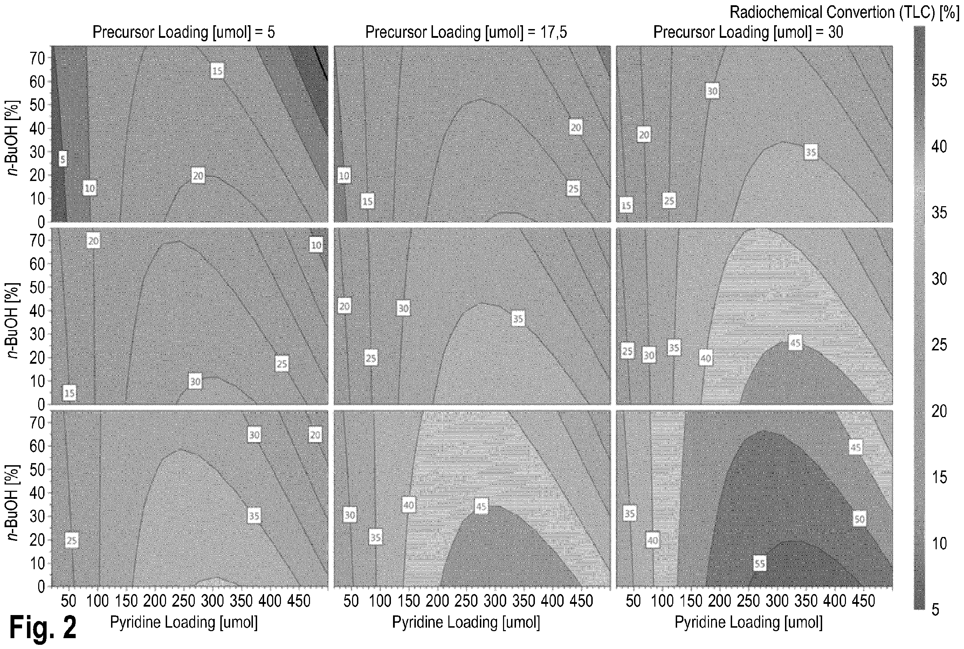

The use of larger amounts of precursor was found to have a positive effect on reaction performance, while smaller amounts of the copper mediator (Cu(OTf)₂) were beneficial. A small amount of n-BuOH (5-10%) was also found to provide a small increase to reaction performance. It is though that n-BuOH increases the rate of the CMRF transmetalation step. Factor interactions (where the setting of factor effects the behavior of another) were found between pyridine and the precursor amount as well as pyridine and n-BuOH. The effect of pyridine on reaction performance was found to possess strong quadratic (py², curved) behavior. Optimal reaction conditions of 30 µmol **7a,** 300 µmol pyridine, and 5 µmol Cu(OTf)₂ in DMA with 10% n-BuOH, were chosen from the response surface plot generated from the regression model (**Fehler! Verweisquelle konnte nicht gefunden werden.**C; see Figure 2 for a full 4D response surface plot.) These conditions were validated manually using full batches of processed [¹⁸F]TBAF and were able to produce protected (±)[¹⁸F] talazoparib-SEM with 58 ± 7.4 % RCY. These results align with DoE model and afford the product in yields acceptable for automation and enantiomeric resolution. Quantitative deprotection to (±) [¹⁸F] talazoparib (>95% conversion according to HPLC) was achieved with 6 M HCI at 100 °C for 15 min.

The optimized CMRF radiosynthesis was translated onto two separate synthesis platforms: An Elixys Flex/Chem automated radiosynthesizer coupled to a Pure/Form purification and formulation module (Sofie biosciences, USA), and a FX_{N} Pro (GE, Sweden). The Elixys Flex/Chem platform is a cassette-based system (up to 3) and is able to perform complex multi-reactor radiosynthesis, while the Pure/Form module is equipped with two semipreparative HPLC injection loops (5 ml) and up to 3 selectable HPLC columns. This setup would therefore allow for the sequential HPLC based purification and enantiomeric resolution required for the synthesis of [¹⁸F] talazoparib. The Tracerlab FX N Pro is a fixed fluid path system with a single HPLC injection loop and column; therefore, the purified product racemate must be transferred to a secondary external HPLC system (in this case an Elixys Pure/Form) for enantiomeric resolution. While the exact technical details differ between the two synthesis modules (the automated radiosynthesis using both systems are described in detail in the SI), the automated synthesis of [¹⁸F] talazoparib follows the same general procedure irrespective of which module is used (**Fehler! Verweisquelle konnte nicht gefunden werden.**).

The DoE optimized reaction mixture was added to the processed dry [¹⁸F]TBAF and reacted at 120 °C for 20 minutes. This was followed by the removal of the SEM protecting group with 6M HCl 120 °C for 10 min. The pH of the reaction mixture was then adjusted to pH 5-7 with the addition of NaOH (2M). The reaction mixture was then diluted and passed over an HLB SPE cartridge (Waters), trapping the product (±)[¹⁸F] talazoparib and removing residual salts and unreacted [¹⁸F] fluoride. The product is then eluted from the HLB cartridge with acetonitrile (1 ml) and reformulated with HPLC buffer for purification in the first HPLC step (C-18 reverse-phase). The product HPLC peak is isolated (< 5 mL) and this solution is then transferred to a second HPLC injection loop for enantiomeric resolution using a semipreparative CHIRALPAK IB-N5 (5µm, 10 x 250 mm) column operating under reverse-phase conditions. The solvents and columns used for the chiral separation HPLC step were carefully chosen to be directly compatible with the conditions of the first HPLC step. The product enantiomer is then isolated, diluted, and trapped on a second HLB cartridge. The product [¹⁸F] talazoparib is then eluted from the cartridge and formulated for injection with ethanol (5%) and phosphate buffered saline (PBS.) This procedure was able to produce enantiomerically enriched (>95% ee) [¹⁸F] talazoparib with 12 ± 1.5% RCY (activity yield = 4-6% over 120 min); molar activity = 45-176 GBq/µmol. Product identity, chemical and radiochemical purity, and molar activity was determined using a CHIRALPAK IB-N5 (5 µm, 4.6 X 150 mm) analytical HPLC against the commercially available non-radioactive standard compound.

*¹⁸F Processing.* ¹⁸F processing may be carried out by using known procedures. For instance, [¹⁸F]Fluoride, delivered from the cyclotron in an aqueous solution, was passed over a preconditioned QMA-OTf cartridge. (The QMA cartridge was conditioned using 10 ml KOTf solution in water (90 mg/ml), followed by air (10 ml), followed by water (10 ml), followed by air (10 ml)). The [¹⁸O]H₂O (or cyclotron target wash water) was collected in a separate vial for recycling, and a stream of argon was used to blow any residual water off the QMA cartridge. Once "air dry", the [¹⁸F] fluoride was eluted from the QMA as [¹⁸F]TBAF, using a solution of TBAOTf (10 mg) in methanol (1 ml). The resulting solution of [¹⁸F]TBAF in methanol was then either distributed into aliquots for DoE optimization experiments or used as a full batch for tracer productions. The [¹⁸F]TBAF was added into a reactor vessel, and the methanol was removed by evaporation under a stream of argon gas to afford dry [¹⁸F]TBAF without the need for extended azeotropic drying.

To dry [¹⁸F]TBAF is a single used glass reactor vessel was added a premade solution containing the required quantities of the precursor for radiolabeling (**7a-c**), Cu(OTf)₂, pyridine, DMA, and n-BuOH (total reaction volume of 700 µl). The reaction mixture was then stirred for at 120 °C for 10 minutes before being quenched with HCl (0.25 M, 700 µl) or reacted further with 6M HCl. Reaction performance was evaluated using radioTLC, which was read out using a Cyclone Plus storage phosphor imaging system (*PerkinElmer,* Waltham, Massachusetts, USA). Compound identity was confirmed by analytical radioHPLC against the non-radioactive standard compound.

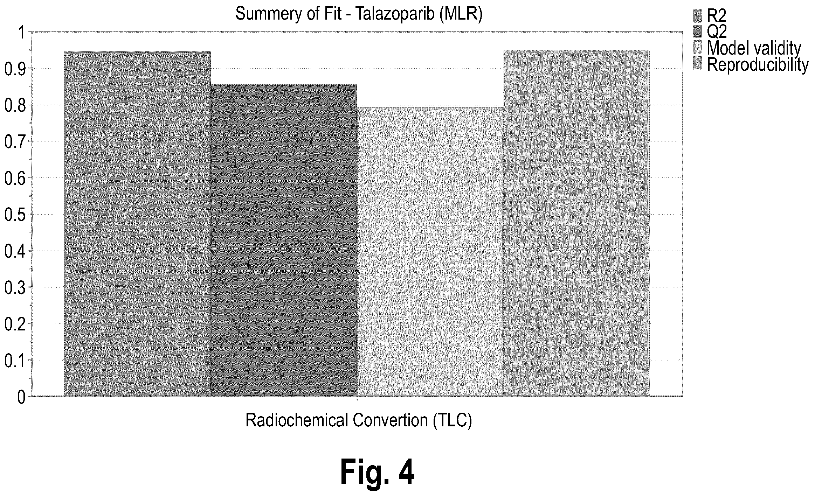

Fig. 3 shows the general automated procedure for the radiosynthesis. From Fig. 4 the summary statistics for D-Optimal DoE for CMRF of [¹⁸F] talazoparib are derivable.

A computer-generated D-optimal DoE (design of experiments) study was designed and analyzed using *MODDE Go 12* (Sartorius, Germany). The D-optimal DoE study was performed to maximize the radiochemical yield of the CMRF step of the [¹⁸F] talazoparib radiosynthesis across four experimental factors: The precursor load (*Pre,* 5-30 µmol), the copper-mediator load (*Cop*, 5-40 µmol), the pyridine load (*Py*, 20-500 µmol), and the % of *n*-BuOH co-solvent (*Bu*, 0-75%). The resulting worksheet table (table 1) was used to calculate the required reactants, reagents, and solvents for each experiment, and the study was performed over 4 days using four cyclotron target washes. All DoE experiments were carried out in a randomized order. All reactions (tests and tracer productions) were carried out using a racemic mixture of protected (**7a**) precursor. In all cases, [¹⁸F]fluoride was processed into [¹⁸F]TBAF via the general procedure described above.

The response (Y) was the %RCY of the labeling reaction and was measured by radio TLC (100% ethyl acetate development solvent). Selected runs were analyzed via radio HPLC against a non-radioactive standard to confirm compound identity.

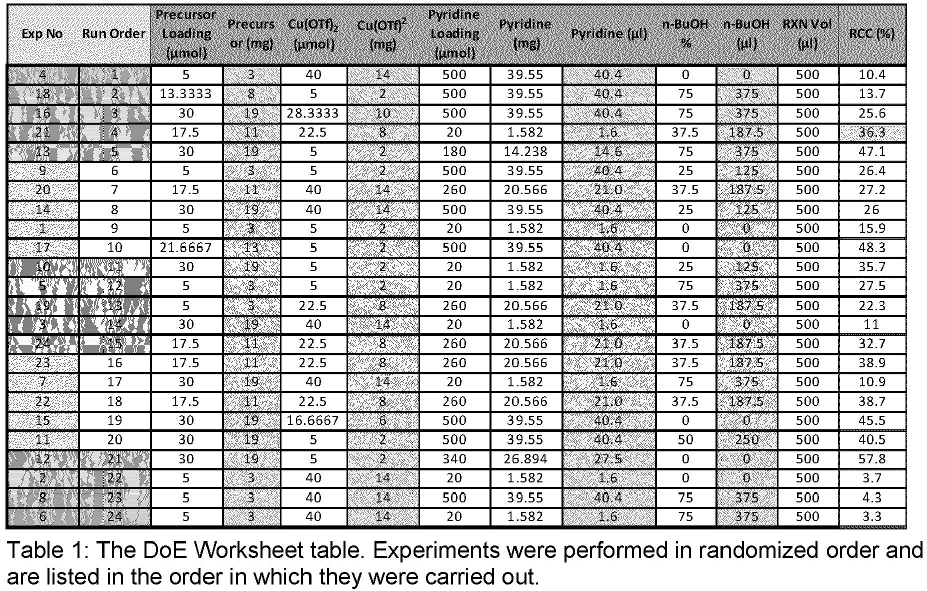

The detailed automated radiosynthesis of [¹⁸F] talazoparib, using both the Elixys (Sofie bioscience, USA) and FX N pro (GE, Sweden) systems is described in detail in hereinafter.

The general procedure is as follows: [¹⁸F]Fluoride in [¹⁸O]water was delivered into the module directly from the cyclotron where it was trapped on a QMA cartridge (OTf-form). The [¹⁸O]water was collected for recycling. The ¹⁸F was then eluted with a solution of tetrabutylammonium triflate (TBAOTf) in methanol (1 ml), which was evaporated to dryness under a stream of argon to afford base-free [¹⁸F]TBAF. The DoE optimized reaction mixture, consisting of **7a** (19 mg, 30 µmol), Cu(OTf)₂ (3 mg, 5 µmol), and pyridine (24 µl, 300 µmol) in 700 µl DMA with n-BuOH (10%), was then added to the [¹⁸F]TBAF reacted at 120 °C for 20 min. After cooling the reactor vessel to ambient temperature HCI (6M, 700 µl) was added to the mixture and reacted at 120 °C for 10 min. The reaction vessel was again cooled, and the reaction was quenched with the addition of NaOH (2 M, 2.1 ml). The mixture was diluted with water (<15% organic solvents) and passed over an HLB SPE cartridge, trapping the product. The product was then eluted into a 5 ml vessel with acetonitrile (1 ml) and HLPC buffer (4 ml) and this solution was transferred to the first HPLC injection loop (5ml). The product racemate was then isolated from the reaction mixture using reverse-phase HPLC (C-18 Luna (10 µm, 10 x 250 mm)) via an isocratic method using water (0.1% TFA):acetonitrile (72:28). The radioactive fraction was collected in seal v-vial that was subsequently pressurized to load the solution onto a second HPLC injection loop. The purified racemic product was then injected on to a semipreparative CHIRALPAK IB-N5 (5 µm, 10 x 250 mm) HPLC column for enantiomeric resolution. The radioactive fraction corresponding to [¹⁸F] talazoparib was collected in a dilution reservoir, the contents of which was subsequently passed over an HLB cartridge. The product [¹⁸F] talazoparib was eluted from the cartridge with ethanol (0.5 ml) and reconstituted with PBS (4.5 ml).

*Quality control.* Product identity was confirmed with analytical CHIRALPAK IB-N5 (5 µm, 4.6 x 150 mm) HPLC against a commercially acquired non-radioactive standard samples of talazoparib and its biologically inactive enantiomer LT-674. The molar activity of the radiotracer was calculated from the analytical HPLC UV signal using a calibration curve generated from the serial dilution of a standard sample of talazoparib.

The automated radiosynthesis of [¹⁸F] talazoparib may feature the following process steps: 1) Radiosynthesis of (±) [¹⁸F] talazoparib. 2) Semi-preparative HPLC of (±)[¹⁸F] talazoparib. 3) Enantiomeric separation of [¹⁸F] talazoparib and [¹⁸F]LT-674 via Semi-preparative Chiral-PAK HPLC . 4) Tracer concentration and formulation. It will be clear, however, that this segregation is for better understanding and may be employed in a different manner.

All four steps could be performed using an Elixys FLEX/CHEM synthesizer coupled to a PURE/FORM concentration and formulation module. Alternatively, steps 1) and 2) could be performed using a FX N Pro (GE), after which the compound was transferred to an external purification and Formulation module for steps 3) and 4).

The radiosynthesis of [¹⁸F] talazoparib was performed using an Elixys Flex chem with three Elixys cassettes and three 5 ml v-vial reactor vessels. Each cassette was loaded with prefilled reagent vials in accordance with the table 2. A pear-shaped distillation flask was used as a dilution reservoir and was situated between cassettes 1 and 2. The cassettes were set up and connected as shown in Fig. 5. Cassette 1 is for radiosynthesis and deprotection. Cassette 2 is for formulation for HPLC injection. HPLC Col. 1: C-18, solvent: water (0.1% TFA): MeCN; (solvent ratio 72:78). HPLC Col. 2: IB-N5, Water (0.1% TFA): MeCN; (solvent ratio 60:40). The syringe pump is for 1. water, 2. ethanol, 3. PBS.The PURE/FORM syringe pump was primed before each synthesis.

**Table 2: Synthesis reagents, cartridges, and eluents, and their corresponding cassette positions.**

| **Cassette and Reagent Position** | **Reagent** |
|---|---|
| Cassette 1; Position 1: QMAEluent | TBAOTfin Methanol (10 mg/ml) 1 ml |
| Cassette 1; Position 2: Reaction Mixture | **7a** (19 mg); **Cu(OTf)₂** (3 mg); **Pyridine** (24 µl); **DMA** (606 µl); ***n*** - **BuOH** (70 µl) |
| Cassette 1; Position 3 | HCl 6M (700 µl) |
| Cassette 1; Position 4 | Ammonium Formate Buffer (25 mM, 1.5 ml) + NaOH (6M, 300 µl) |
| Cassette 1; Position 5 | Ammonium Formate Buffer (25 mM, 1.5 ml) + NaOH (6M, 300 µl) |
| Cassette 1; Position 6 | Acetonitrile(1 ml) |
| Cassette 2; Position 1 | Water (0.1% TFA) (2 ml) |
| Cassette 2; Position 2 | Water (0.1% TFA) (2 ml) |
| Dilution Reservior 2 (PURE/FORM) | Water (60 ml) |
| Cassette 1; Cartridge Loop 1 (BLUE) | QMA (KOTf Preconditioned) |
| Cassette 1; Cartridge Loop 2 (RED) | HLB (Conditioned EtOH (2 ml), water (2 ml)) |
| PURE/FORM SPE Loop | HLB (Conditioned EtOH (2 ml), water (2 ml)) |
| HPLC Eluent A | Water (0.1% TFA) |
| HPLC Eluent B | Acetonitrile |
| HPLC Column 1 | C-18 Luna (10 µm, 10 mm x 250 mm) |
| HPLC Column 2 | CHIRALPAK IB-N5 (5 µm, 10 mm x 250 mm) |
| PURE/FORM Syringe Pump "water" | Water (40 ml) |
| PURE/FORM Syringe Pump "Ethanol" | Ethanol (20 ml) |
| PURE/FORM Syringe Pump "Saline" | PBS (40 ml) |

The Elixys sequence for the radiosynthesis of [¹⁸F] talazoparib (listed as Elixys unit operations) is as follows: Step 1: Trap Isotope (¹⁸F delivered directly from the cyclotron, over the QMA cartridge in Position A). Step 2: Elute Isotope (Cassette 1: Position 1: QMA Eluent). Step 3: Evaporate (90 °C, 4-7 minutes or until no liquid is observed through reactor camera). Step 4: Add Reagent (Cassette 1: Position 2: Reaction Mixture). Step 5: Move Reactor (Blow 20 ml of air into the reactor vessel with a syringe fitted with a long needle). Step 6: React (120 °C, 20 min). Step 7: Add Reagent (Cassette 1: Position 3: HCI (700 µl)). Step 8: React (100 °C, 15 min). Step 9: Add Reagent (Cassette 1: Position 4: water (2 ml)). Step 10: Transfer (Out to collection vial (dilution reservoir 1)). Step 11: Add Reagent (Cassette 1: Position 5 water, (3 ml)). Step 12: Transfer (Out to collection vial (dilution reservoir 1)). Prompt 13: Remove the needle from cassette 1 and the vent needle from the dilution reservoir. Step 14: Trap Isotope (From external vial, over the HLB (*Waters*) cartridge in position B, 8 minutes). Step 15: Elute Isotope (Cassette 2: Position 1: acetonitrile (1 ml)). Step 16 and 17: Add Reagent (Cassette 2: Positions 2 & 3: HPLC Aqueous phase (4 ml)). Step 18: Transfer (Cassette 2 to PURE/FORM Loop 1 (manual injection)). When the transfer is complete (fluid detector reads "No Fluid"), trigger HPLC injection manually. Step 19: Semiprep HPLC 1. Column: C-18 Luna (10 µm, 10 x 250 mm). HPLC Eluent (Isocratic): 28 % Acetonitrile; 72 % Water (w/ 0.1% TFA); 6 ml/min. The product radio peak elutes at approximately 10:30-13 min and is cut into a sealed v-vial (10 ml) fitted with a vent, a connection to cassette 3, and long needle pushed to bottom of the vial for transfer to HPLC loop 2. Prompt 20: After the radioactive fraction has been collected, remove the vent needle form the HPLC transfer vial. Step 21: Transfer (Cassette 3 (only provides gas pressure) to PURE/FORM Loop 1 (manual injection)). Step 22: Semiprep HPLC 2. Column: CHIRALPAK IB-N5 (5 µm, 10 x 250 mm) fitted with CHIRALPAK IB-N5 (5 µm, 10 x 20mm) column guard. HPLC Eluent (Isocratic): 40 % Acetonitrile; 60 % Water (w/ 0.1% TFA); 5 ml/min. The product enantiomer peak elutes at approximately 9-10 min (see attached HPLC Trace) and is cut into the PURE/FORM Dilution reservoir (Containing 60 ml water) for SPE reformulation. The Inactive enantiomer elutes at 14:30-15:30 min and can be isolated using the Elixys fraction collector if need be. Step 23: Formulation. The dilution reservoir contents are passed over an HLB cartridge, trapping the purified [¹⁸F]olaparib. Ethanol (0.5 ml) is then used to elute the radiotracer into a product vial. PBS (4.5 ml) is then used to reconstitute the tracer in the product vial.

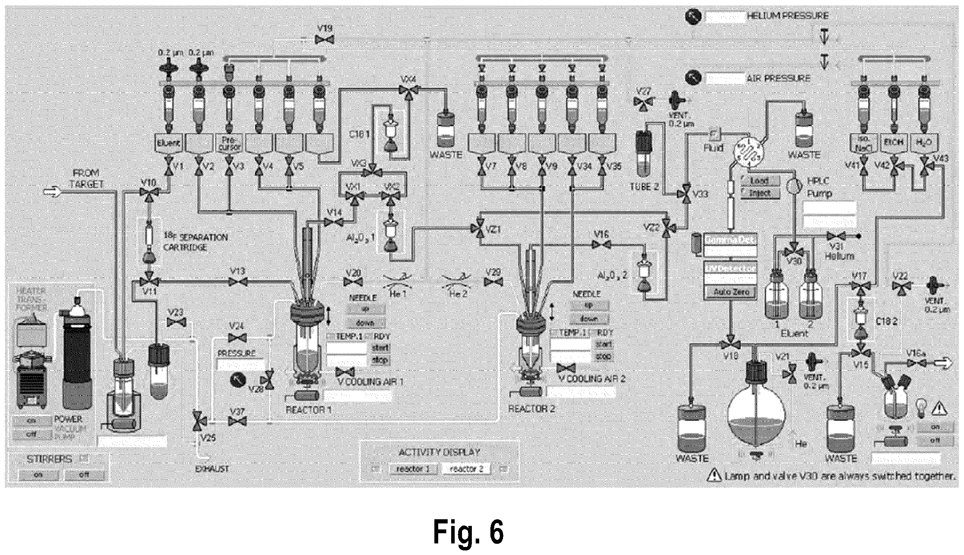

Semi-automated Radiosynthesis of [¹⁸F] talazoparib is performed using a GE FX N Pro Radiosynthesis System and Elixys PURE/FORM module. The FX N Pro was set up as depicted in Fig. 6. Reagent vials were filled as described in the table 3 below.

**Table 3: Synthesis reagents, cartridges, and eluents and their corresponding positions.**

| **Reagent Position** | **Reagent** |
|---|---|
| Vial 1: QMA Eluent | TBAOTf in Methanol (10 mg/ml) 1 ml |
| Vial 2: Reaction Mixture | **OLA-Bpin** (7 mg); **[Cu(OTf)₂(Impy)₄]** (18 mg); DMI (700 µl) |
| Vial 3: | TFA (700 µl) |
| Vial 4: | Ammonium Formate Buffer (25 mM, 12.5 ml) |
| Vial 5: | Methanol Wash (0.5 ml) |
| Vial 6: | Acetonitrile (1 ml) |
| Tube 2: For HPLC injection | Ammonium Formate Buffer (25 mM, 3.5 ml) |
| Vial 12: Formulation | PBS (4.5 ml) |
| Vial 13: Formulation | EtOH (0.5 ml) |
| Vial 14: Formulation | Water (4 ml) |
| Dilution Reservior (Crystal Ball) | Water (60 ml) |
| QMA Cartridge Slot: QMA | QMA (KOTf Preconditioned) |
| C-18 Cartridge Slot 1: HLB | HLB (Conditioned EtOH (2 ml), water (2 ml)) |
| C-18 Cartridge Slot 2: HLB | HLB (Conditioned EtOH (2 ml), water (2 ml)) |
| HPLC Eluent A: (For purification) | Ammonium Formate Buffer (25 mM) |
| HPLC Eluent B: (For column flush) | Acetonitrile |
| HPLC Column | C-18 Luna (10 µm, 10 mm x 250 mm) |

The synthesis protocol is shown hereinafter. At the end of bombardment (EOB), ¹⁸F was delivered from the cyclotron into a delivery vial contained within the FX N pro. The contents of the vial were then drawn over the QMA cartridge by vacuum, and the [¹⁸O]water was collected in a separate waste vial for recycling. The ¹⁸F was then eluted from the QMA with TBAOTf (10 mg) in methanol (1 ml) into the reactor. The walls of the reaction vessel were then washed with methanol (0.5 ml) from vial 5, and the methanol was removed by evaporation at 90 °C for 5 minutes under vacuum and a stream of helium. The reaction mixture was drawn into the reaction vessel with vacuum from vial 2. As vial 2 was open to the air, air was thus also drawn into the reaction vessel over 1 minute. The reactor was sealed and heated to 120 °C for 20 minutes, after which it was cooled to 45 °C, and the contents of vial 3 (TFA, 700 µl) were pushed into the reactor using helium carrier gas. The reactor was then again heated to 120 °C for 15 minutes to allow for the removal of the SEM protecting group. 12.5 ml of 25 mM ammonium formate buffer (from vial 4) was then added to the reaction mixture with stirring. The reactor needle was lowered to the bottom of the reactor, and the contents were pushed over the first HLB cartridge to trap the crude product. The crude product was then eluted into tube 2 (containing ammonium formate buffer, 3.5 ml, 25 mM) with acetonitrile (1 ml, from vial 6). The contents of tube 2 were then loaded onto the HPLC injection loop (5 ml) and injected onto the HPLC column for purification using eluent A. The product radio peak appeared at 10-12 minutes and was cut into the large dilution reservoir. The contents of the dilution were then stirred and passed over the second HLB cartridge into the waste. The product, which was trapped on the HLB cartridge, was washed with water (4 ml, from vial 14), after which it was eluted with ethanol (0.5 ml) into the product collection vial. The product was finally reconstituted with PBS (4.5 ml). The final tracer solution was pushed out into a sterile product delivery vial to afford a solution of [¹⁸F] olaparib (5.4 ± 1.6 %AY; 9.3 ± 3.3 %RCY; synthesis time 90 min) in PBS with 10% ethanol.

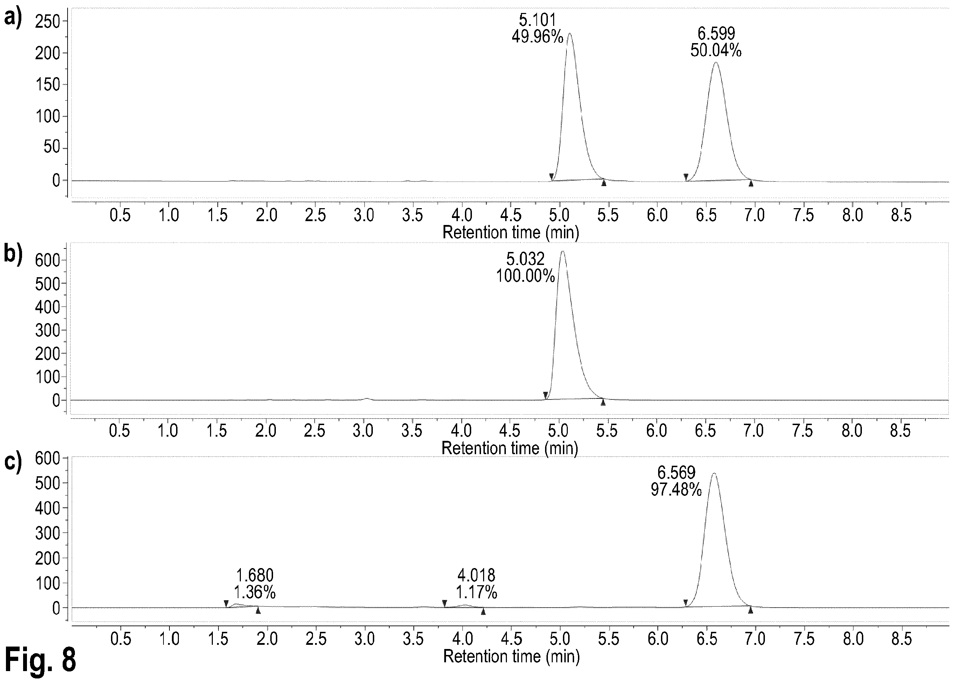

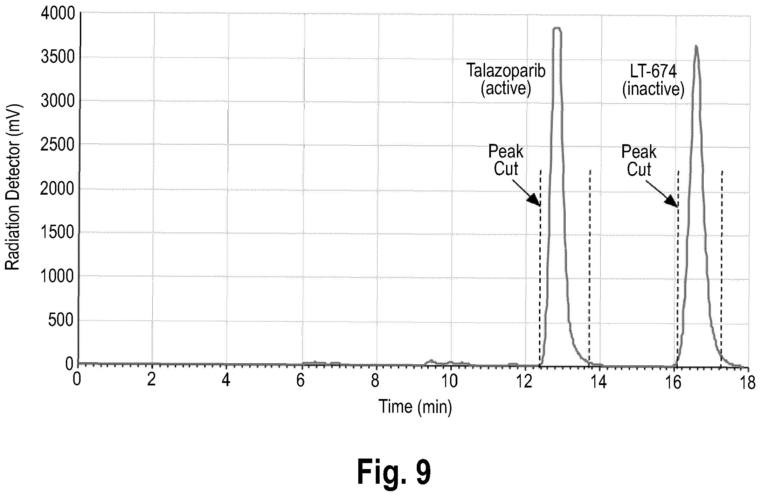

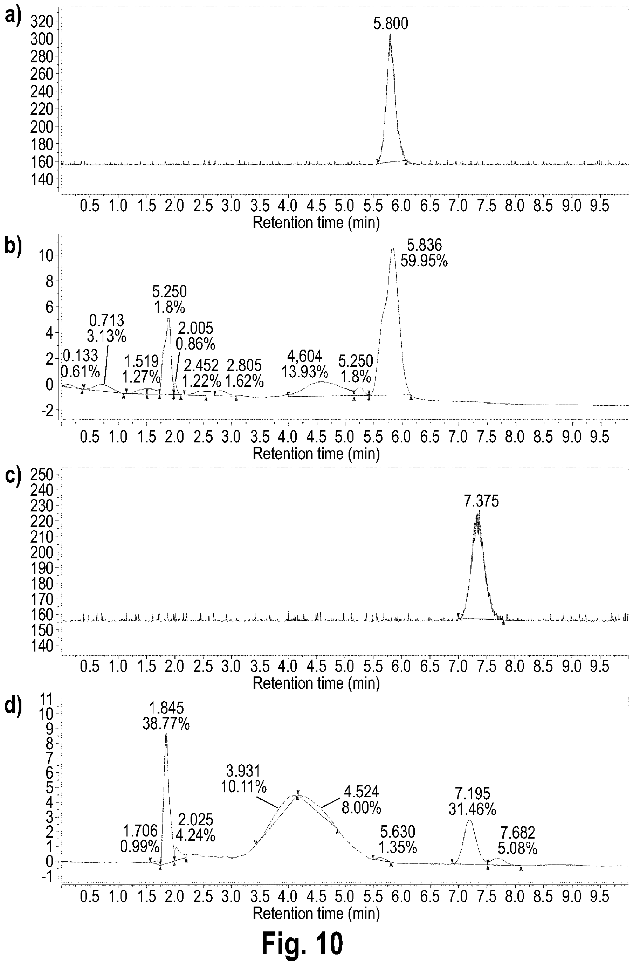

Figs. 8 to 10 show RadioHPLC of the different compounds.

The synthesis of the compound according to the invention is illustrated by the following clauses.
1. A compound of the formula 10a and/or 10b 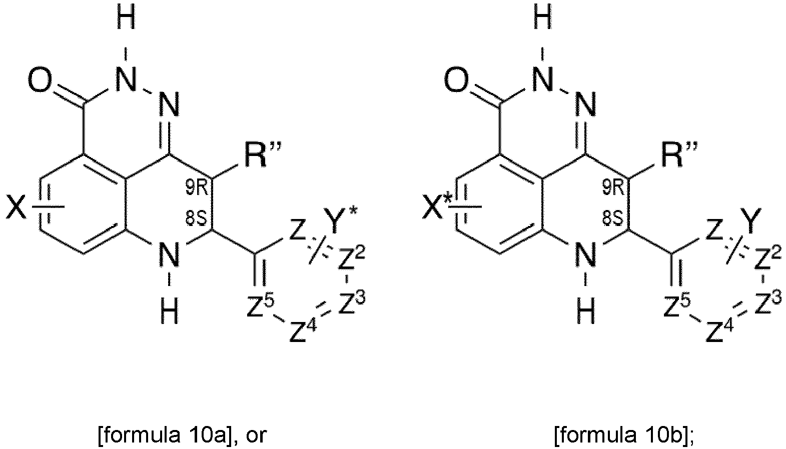 or
   a precursor of the formula 5c 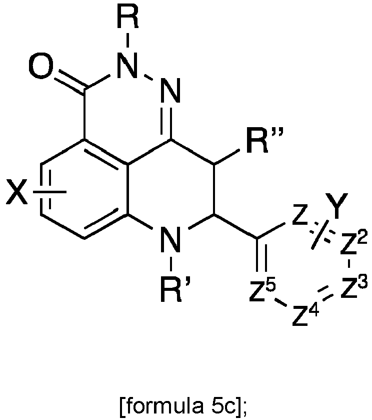 wherein
   R and R' are amine protecting groups;
   R" is a heteroaromatic or an aromatic residue of a molecular weight of 120 g/mol or less;
   X and Y are independently selected from the group consisting of H, one or more leaving groups for [¹⁸F] fluorination, and a saturated or unsaturated alkyl residue of a molecular weight of 80 g/mol or less, provided that at least one leaving group for [¹⁸F] fluorination is present;
   X* and Y* are independently selected from the group consisting of H, [¹⁸F], one or more leaving groups for [¹⁸F] fluorination and a saturated or unsaturated alkyl residue of a molecular weight of 80 g/mol or less, provided that at least one [¹⁸F] is present;
   z and z²⁻⁵ are independently selected from the group consisting of C and N; an enantiomeric ratio (er) at a position C8 and/or a position C9 of the precursor is ≥ 1.
2. A method for producing a compound of the formula 10a and/or 10b 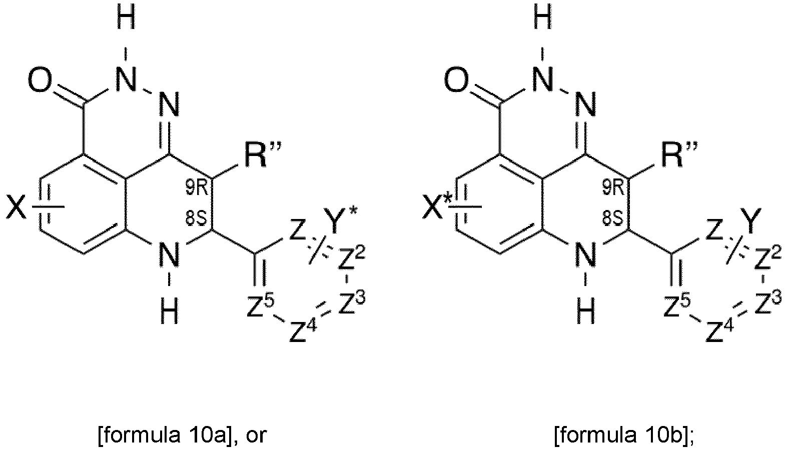 comprising the steps of:
   (A) providing a precursor of the formula 5c 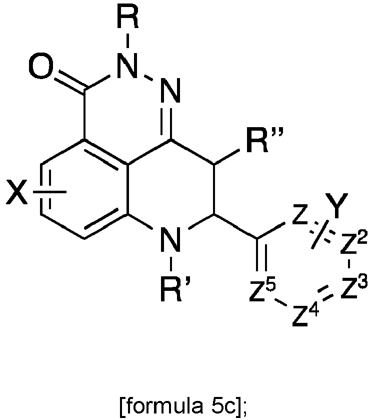
   (B) reacting the precursor of the formula 5c with an [¹⁸F] radiofluorination agent to obtain a [¹⁸F] fluorinated compound of the formula 8a and/or 8b, 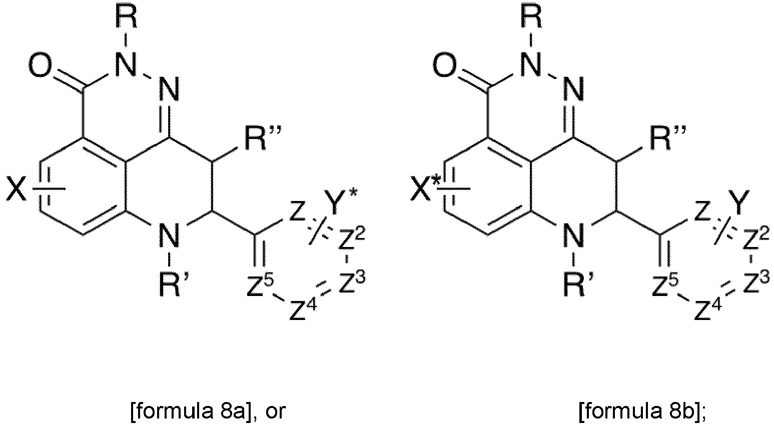
   (C) de-protecting the [¹⁸F] fluorinated compound of the formula 8a and/or 8b to obtain a compound of the formula 9a and/or 9b 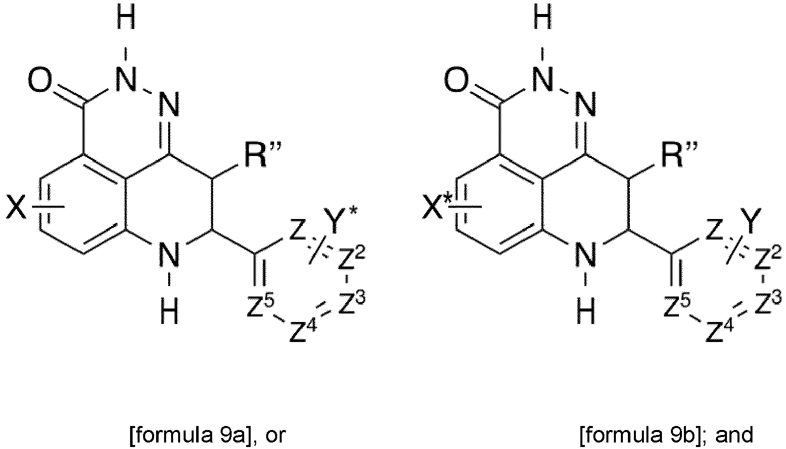
   (D) subjecting the compound of the formula 9a and/or 9b to purification and optionally enantiomeric resolution to obtain the compound of the formula 10a and/or 10b,
      wherein
      R and R' are amine protecting groups;
      R" is a heteroaromatic or an aromatic residue of a molecular weight of 120 g/mol or less;
      X and Y are independently selected from the group consisting of H, one or more leaving groups for [¹⁸F] fluorination, or a saturated or unsaturated alkyl residue of a molecular weight of 80 g/mol or less, provided that at least one leaving group for [¹⁸F] fluorination is present;
      X* and Y* are independently selected from the group consisting of H, [¹⁸F], one or more leaving groups for [¹⁸F] fluorination and a saturated or unsaturated alkyl residue of a molecular weight of 80 g/mol or less, provided that at least one [¹⁸F] is present;
      z and z²⁻⁵ are independently selected from the group consisting of C and N;
      an enantiomeric ratio (er) at a position C8 and/or a position C9 of the precursor is ≥ 1.
3. The compound of clause 1 or the method of clause 2, wherein the amine protecting group at R and R' is independently selected from the group consisting of Trimethylsilylethoxymethyl, *t*-Butyl carbamate, Benzyloxy carbamate, and Methoxymethyl acetal.
4. The compound of clause 1 or 3 or the method of any of clauses 2-3, wherein the heteroaromatic residue is selected from the group consisting of Me-1,2,4-triazol-5-yl, 4-Me-1,2,4-triazol-3-yl, 4-pyridyl, 3-pyridyl, 2-pyridyl, 1-Me-imidazol-2-yl, 1-Me-pyrazol-5-yl, wherein the aromatic residue is phenyl.
5. The compound of any of clauses 1 or 3-4 or the method of any of claims 2-4, wherein z is N and z²⁻⁵ are C; z² is N and z, z³⁻⁵ are C; or z³ is N and z, z², z⁴⁻⁵ are C.
6. The compound of any of clauses 1 or 3-5 or the method of any of clauses 2-5, wherein the leaving group for [¹⁸F] fluorination is selected from the group consisting of Boronic acid, Boronic acid ester, lodonium salt, lodonium ylid, Trialkylstannane, Nitro, Trialkyl ammonium salts, and Sulfonate esters, such as OTs, OMs, ONs, OBs, or OTf.
7. The compound of any of clauses 1 or 3-6 or the method of any of clauses 2-6, wherein the radiofluorination agent is a nucleophilic [¹⁸F] radiofluorination agent, preferably wherein the nucleophilic [¹⁸F] radiofluorination agent is [¹⁸F] TBAF.
8. The compound of any of clauses 1 or 3-7 or the method of any of clauses 2-7, wherein the compound of the formula 10a is [¹⁸F] talazoparib, or
   the compound of formula 10b is (8S,9R)-5-[¹⁸F]-8-(4-fluorophenyl)-2,7,8,9-tetrahydro-9-(1-methyl-1H-1,2,4-triazol-5-yl)- 3H-pyrido[4,3,2-de]phthalazin-3-one.
9. The method of clauses 2, including the step of preparing the precursor of the formula 5c by
   (i) reacting a compound of the formula 5d and/or 5e 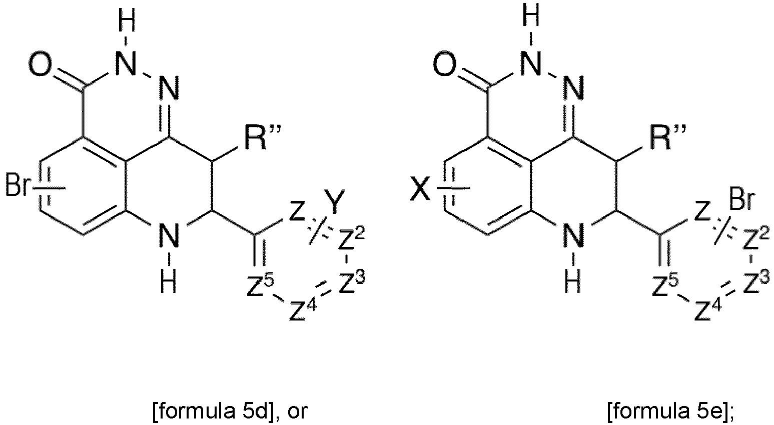 with a trimethylsilylethoxymethyl salt, preferably trimethylsilylethoxymethyl chloride; and
   (ii) derivatizing to the corresponding boronic acid pinacol esters of the formula 7d and/or 7e 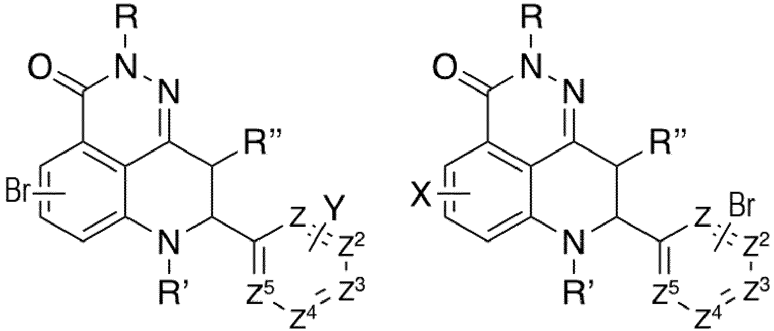 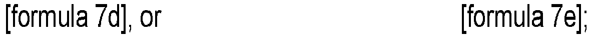 wherein R' is trimethylsilylethoxymethyl, R" is trimethylsilylethoxymethyl or H, and the leaving group for [¹⁸F] fluorination is boronic acid pinacol ester or tributylstannane.
10. The method of clause 9, wherein the compound of the formula 5d is (±)-5-Fluoro-8-(4-bromophenyl)-2,7,8,9-tetrahydro-9-(1-methyl-1*H*-1,2,4-triazol-5-yl)-3*H*-pyrido[4,3,2-de]phthalazin-3-one, or the compound of the formula 5e is (±)-5-bromo-8-(4-fluorophenyl)-2,7,8,9-tetrahydro-9-(1-methyl-1*H*-1,2,4-triazol-5-yl)-3*H*-pyrido[4,3,2-*de*]phthalazin-3-one.
11. The method of any of clauses 2-10, wherein purification of the compound of the formula 9 of step (D) includes subjecting the compound of the formula 9 to solid phase extraction with a sorbent for polar analytes to obtain a purified compound of the formula 9.
12. The method of clause 11, wherein enantiomeric resolution of the compound of the formula 9 of step (D) includes subjecting the purified compound of the formula 9a or 9b to chiral HPLC or chiral SFC to obtain the compound of the formula 10a and/or 10b.
13. The method of any of clauses 2-11, wherein the step (B) of reacting the precursor of the formula 5c with an [¹⁸F] radiofluorination agent includes copper mediated radiofluorination chemistry that is optimized with respect to the radiochemical yield of the (±)¹⁸F fluorinated compound of the formula 8a or 8b.
14. Method for preparing a diagnostic agent comprising the steps of:
   providing the compound of formula 10a and/or 10b according to any of clauses 1 or 3-8; and
   formulating the compound with a pharmaceutically acceptable carrier and/or a solvent and, if applicable, further pharmaceutical excipients,
   preferably wherein the diagnostic agent is designated for a use within the positron emission tomography.

The present inventors adapted the synthesis of the precursor from the synthetic route employed by Wang *et al.* for the synthesis of talazoparib (Wang, B.; Chu, D.; Feng, Y.; Shen, Y.; Aoyagi-Scharber, M.; Post, L. E., Discovery and Characterization of (8S,9R)-5-Fluoro-8-(4-fluorophenyl)-9-(1-methyl-1H-1,2,4-triazol-5-yl)-2,7,8,9-te trahydro-3H-pyrido[4,3,2-de]phthalazin-3-one (BMN 673, talazoparib), a Novel, Highly Potent, and Orally Efficacious Poly(ADP-ribose) Polymerase-1/2 Inhibitor, as an Anticancer Agent. J Med Chem 2016, 59 (1), 335-57). It will be appreciated, however, that also other synthetic routes may be used as starting points for the provision of the precursor of formula 5c.

In particular, 1-methyl-1H-1,2,4-triazole was formylated to 1 via the published procedure (W. Hungate; Tae-Seong Kim; Richard T. Lewis; Longbin Liu; Julia Lohman; Mark H. Norman; Michelle Potashman; Aaron C. Siegmund; Stephanie K. Springer; Markian Stec; Ning Xi; Kevin Yang, B. K. A. D. B. S. B. C. M. B. S. B. A. B. D. C. D. D. A. J.-C. H. S. H. R. FUSED HETEROCYCLIC DERIVATIVES AND METHODS OF USE. WO2009/091374). **1** was then condensed with commercially available 6-fluoro-4-nitroisobenzofuran-1(3H)-one in THF in the presence of NEt₃ and Ac₂O to afford **2** in a 53% yield. The lactone **2** was opened to give the keto-ester derivative **3** by warming **2** in methanol with a catalytic amount of acetic acid. In the original synthesis, **3** was then reacted with 4-fluorobenzaldehyde in THF and methanol via reductive cyclization driven by TiCl₃ to afford **4b,** which was cyclized with hydrazine monohydrate to afford (±)talazoparib (**5b**), which was to be used as reference compound. Reacting **3** with 4-bromobenzaldehyde via the same two-step procedure afforded the derivatizable bromide **5a.**

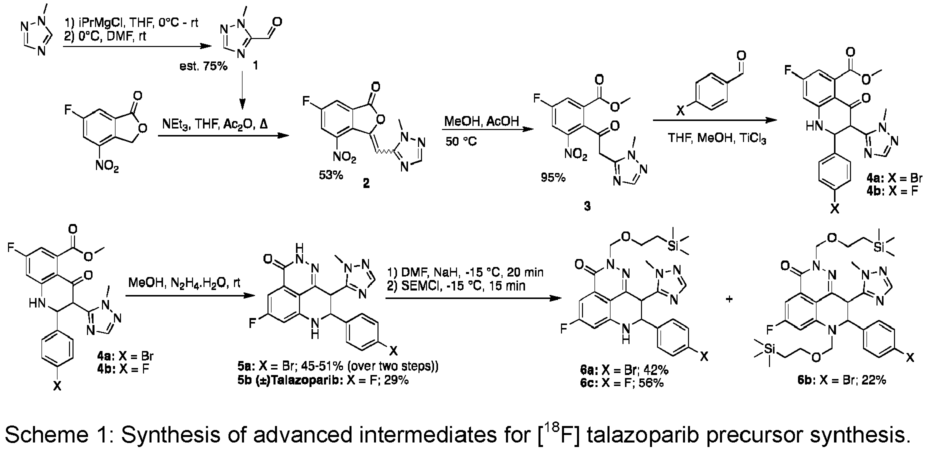

Alternative possibilities for the synthesis of the precursor of the formula 5c as well as the compounds of the 10a and 10b are derivable from scheme 1. The present inventors have found that reacting keto-ester derivative **3** with aromatic aldehydes with different substituents than 4-bromobenzaldehydes influences the overall structure of the compound of the formula 10a. The differently substituted aromatic aldehydes may involve benzaldehyde derivatives, wherein z and z²⁻⁵ are independently selected from the group consisting of C and N, and Y* is selected from the group consisting of H, [¹⁸F], one or more leaving groups for [¹⁸F] fluorination and a saturated or unsaturated alkyl residue of a molecular weight of 80 g/mol or less, provided that at least one [¹⁸F] is present.

It has been furthermore found that reacting **1** with a 4-nitroisobenzofuran-1(3H)-one exhibiting the residue Y* at one or more of the positions C5, C6, and C7 affects the overall structure of the compound of the formula 10b. It will be appreciated that the provision of differently substituted aromatic aldehydes than 4-bromobenzaldehydes as well as a 4-nitroisobenzofuran-1(3H)-one that exhibits the residue Y* at one or more of the positions C5, C6, and C7 is well known to the skilled person.

Employing an aromatic aldehyde that is differently substituted at the phenyl moiety, particularly at any of the positions z²⁻⁵, than 4-bromobenzaldehyde according to scheme 1 and/or a 4-nitroisobenzofuran-1(3H)-one that is differently substituted at one or more of the positions C5, C6, and C7 than 6-fluoro-4-nitroisobenzofuran-1 (3H)-one according to scheme 1 results in the generation of compounds that are different from (±) talazoparib, namely a precursor of the formula 5c.

A protecting group strategy using the trimethylsilylethoxymethyl ether (SEM) protecting group was employed to the compound **5a,** which was protected with trimethylsilylethoxymethyl chloride (SEMCI) using sodium hydride in DMF at 15 °C. These conditions afforded both the mono- (**6a**) and di-SEM (**6b**) protected bromide derivatives. It is clear, however, that the protecting group strategy is not limited to the disclosed compounds.

Compounds **6a** and **6b** were then further derivatized to yield several possible precursors for radiolabeling (Scheme 2). The protection of **5a** favored the formation **6a** over **6b** (ca. 3:1), even under conditions designed to drive the formation of the di-SEM protected derivative. Compounds **6a** and **6b** were converted to their corresponding boronic acid pinacol esters under Miyaura borylation conditions in DMF with KOAc, bis(pinacolato)diboron, and Pd(dppf)Cl₂ as the catalyst (Scheme 2). This afforded the compounds **7a** and **7b** in good to excellent yields. **7a** was purified through recrystallization a shelf-stable and convenient-to-handle white crystalline solid. **7b** however, was purified through chromatography and was much harder to obtain in the high purities required for reliable radiochemistry. Despite being of high chromatographic purity (HPLC-MS), samples of **7b** displayed a complex NMR spectrum, indicative of the presence of multiple conformation isomers. CMRF chemistry has also been well established with aryl stannanes precursors and the present inventors have investigated this variation of the CMRF reaction extensively in our previous work. Thus, the analogous stannylation of **6a** was carried with bis tributyltin to afford the stannyl precursor **7c** as an amorphous glass. It will be readily understood that the above mentioned protecting and deprotecting agents are merely examples and other protecting and deprotecting agents may be used as well.

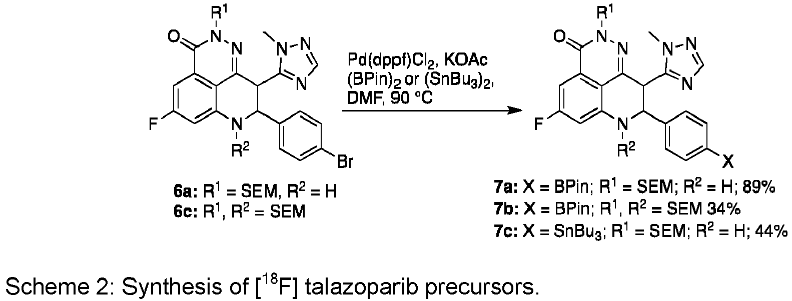

### In Vitro Binding Studies

### Cell culture

Human breast carcinoma cells (HCC1937, ACC513) were purchased from the German Collection of Microorganisms and Cell Cultures (DSMZ GmbH, Braunschweig, Germany) and cultured in Roswell Park Memorial Institute (RPMI) 1640 medium supplemented with 16 % fetal calf serum (FCS), 100 U/ml penicillin and 100 µg/ml streptomycin at 37°C under humid 5 % CO2 atmosphere. Absence of mycoplasma infection was confirmed by PCR analysis in monthly intervals.

### In vitro radiotracer uptake

HCC1937 cells (0.2 × 10⁶) were incubated in 96-well filter plates (MADVN6550, Merck Millipore, Darmstadt, Germany) with 60 µl of a 0.4 MBq/ml radiotracer solution containing either 2.5 µl DMSO as vehicle, 2.5 µl 10 mM olaparib or 2.5 µl talazoparib to a final concentration of 25 µM for blocking. After 30 min of incubation at 37°C, the cells were washed by vacuum filtration of medium through the plate (2×100 µl followed by 2×200 µl), the filters were transferred into tubes using a commercial punch kit (MAMP09608, Merck) and measured in a gamma counter (Wizard 2, Perkin-Elmer, Waltham, MA, USA). Experiments were performed in triplicates and the uptake was quantified as percent of added activity.

### Competition assays

HCC1937 cells (0.2 × 106) were incubated in 96-well filter plates (MADVN6550, Merck Millipore, Darmstadt, Germany) with 40 µl of a 0.4 MBq/ml radiotracer and 20 µl of a 1:2 serial dilution of either talazoparib or olaparib starting with a final concentration of 1µM. After 30 min of incubation at 37°C, the cells were washed by vacuum filtration of medium through the plate (2×100 µl followed by 2×200 µl), the filters were transferred into tubes using a commercial punch kit (MAMP09608, Merck) and measured in a gamma counter (Wizard 2, Perkin-Elmer, Waltham, MA, USA). Experiments were performed in triplicates and the uptake was quantified as percent of added activity.

### In Vivo Evaluation

### PET and MR imaging

All animal experiments were performed according to the German animal welfare act and approved by the local authorities (Regierungsprasidium Tübingen, R3/18). Animals were housed in individually ventilated cages (IVCs, 5 mice per cage) with bedding and enrichment, and food and water was provided *ad libitum.* Animals were kept under isoflurane anesthetic (1.5 % in pure oxygen, 1.5 l/min) during the experiments. 1×10⁷ cells in 1:1 ice-cold Matrigel (Thermo Scientific) / PBS were injected subcutaneously in the right shoulder area of 7 week-old female NOD.CB17-Prkdc^{scid}/J mice (n = 5). After the xenografts reached a suitable size, mice were injected with MBq [¹⁸F] talazoparib and subjected to 1 h dynamic PET imaging (Inveon D-PET, Siemens, Knoxville, TN, USA) with subsequent MR anatomical scans using a 7 Tesla Biospec 70/30 USR (Clinscan, Bruker Biospin MRI GmbH) and a T2-weighted spin echo sequence. The mice underwent a second, 10 min static PET scan 2 h post-injection (p.i.). Mice were sacrificed by cervical dislocation, the collected organs were weighed and the tissue uptake was determined by gamma-counting (WIZARD2, PerkinElmer). PET image reconstruction and correlation with MR images was performed with Inveon Acquisition Workplace and Inveon Research Workplace, respectively, using a user-defined dynamic framing and an ordered subset expectation maximization (OSEM3D) algorithm. Regions of interest (ROIs) were drawn according to the acquired MR images and co-registered with the PET data to obtain time-activity curves (TACs).

### Ex Vivo

### Immunohistochemistry

Immunohistochemistry staining was performed by the Department of Dermatology at the University Hospital Tuebingen, Germany. Sections of paraffin-embedded xenografts were blocked with donkey serum for 30 min and incubated with primary antibody overnight (rabbit anti-PARP (1:50, ab74290, Abcam, Cambridge, UK)). After washing, the sections were incubated for 1 h at room temperature with secondary antibody (donkey anti-mouse IgG Cy3 (1:250, 715-166-151 Dianova)). Nuclei were stained with YO-PRO-1 iodide solution (Y3603, Thermo Scientific) for 5 min; the samples were subsequently mounted with Mowiol (Sigma-Aldrich) and imaged on a LSM 800 microscope (Carl Zeiss).

### Statistical analyses

Statistical analyses were performed with GraphPad Prism 9 using non-parametric t-tests and are represented as mean value ± standard deviation. Blood half-life was calculated using a two-phase decay fit. P-values < 0.05 were considered statistically significant according to the software (*: p < 0.05, **: p < 0.01, ***: p < 0.001, ****: p < 0.0001).

### 2. Results and Discussion

### In Vitro Evaluation

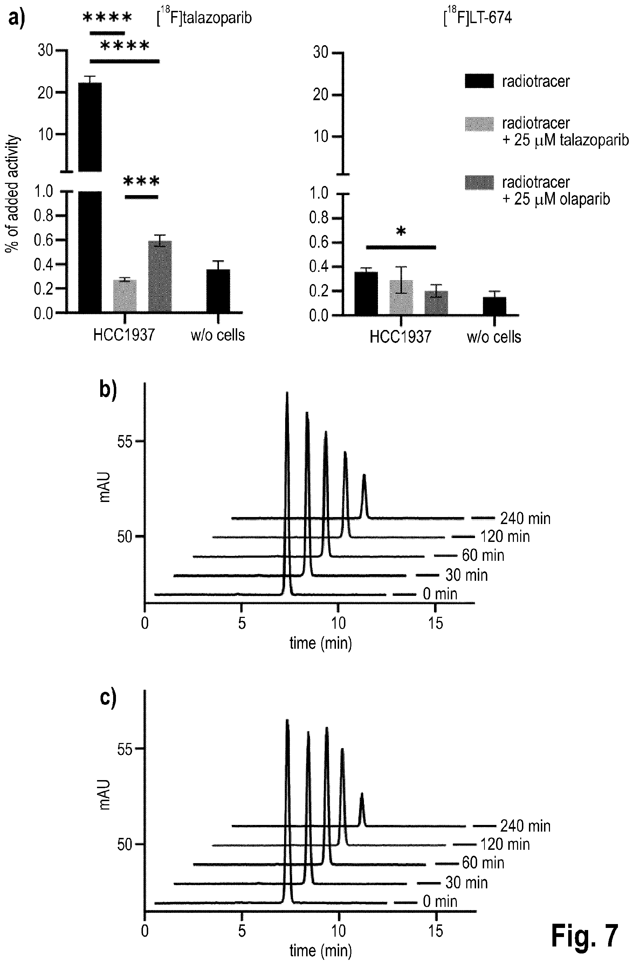

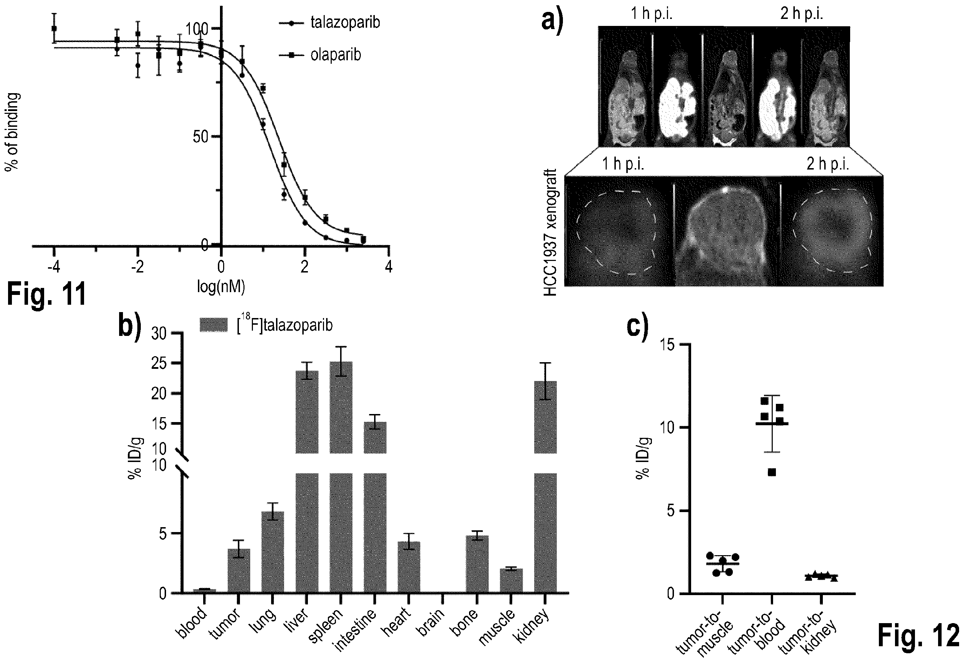

[¹⁸F] talazoparib demonstrated high uptake in HCC1937 cells that was blockable to baseline by both excess olaparib and talazoparib. Notably, talazoparib was observed to block an over 2-fold higher amount of the radioactive signal than olaparib. In contrast, the inactive enantiomer [¹⁸F]LT-674 showed only very low radiotracer uptake although blocking with olaparib resulted in a significantly lower signal (Fig. 7A). A competition assay performed with different concentrations of olaparib and talazoparib resulted in similar curves, however, talazoparib again blocked the signal more effective resulting in a slightly shifted curve (Fig. 11). Stability of [¹⁸F] talazoparib was tested in mouse and human serum and no radiometabolite was detected over a time course of 240 minutes (Fig 7 B).

[¹⁸F] talazoparib thus presented with similar to slightly higher uptake in HCC1937 cells compared to [¹⁸F] olaparib demonstrating high affinity. Since [¹⁸F] talazoparib did not show significantly higher binding to PARP expressing cells compared to [¹⁸F] olaparib, the inventors conclude that only the binding to the catalytic domain can be visualized, as only a slightly better catalytic inhibition of PARP1 by talazoparib over olaparib is reported. Thus, the superior ability of talazoparib to trap PARP1 on the site of the DNA damage seems not to affect the amount of bound compound but might increase the retention time at the binding site. As talazoparib blocked the amount of bound [¹⁸F] talazoparib to a significantly greater extent than olaparib, we theorize that this difference represents a second target of talazoparib besides PARP1.

### In Vivo Evaluation

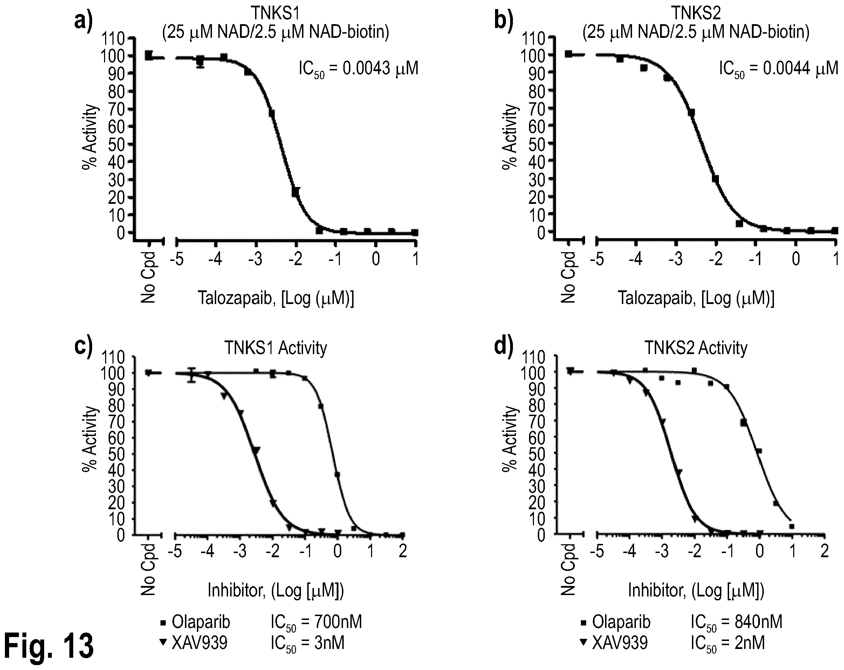

[¹⁸F] talazoparib was further characterized *in vivo* in HCC1937 xenograft-bearing mice showing overall high uptake in the abdomen as well as decent xenograft uptake already 1 h p.i. that further intensified at 2 h p.i. Remarkably, the bone uptake increased as well over time (Fig. 12 A). These findings were further confirmed by the biodistribution analysis 2.5 h p.i. as the overall high uptake peaked in liver, spleen, intestine and kidney (Fig. 12 B). Although the absolute xenograft uptake is considerably high (3.7 ± 0.71 %ID/g), high uptake in muscle tissue resulted in a mean tumor-to-muscle ratio (TMR) of 1.82 ± 0.44 (Fig. 12 C) which is slightly lower than the reported TMR of [¹⁸F] PARPi in the same mouse model (2.95 ± 0.4). However, if relative to blood, the tumor-to-blood ratio (TBR) is outstandingly high with 10.23 ± 1.51 and exceeds the TBR of [¹⁸F] PARPi by a factor of approximately 3 (10.23 ± 1.51), strengthening the hypothesis that the muscle uptake might be caused by binding of [¹⁸F] talazoparib to other enzymes. Potential target candidates are the PARP enzymes tankyrase 1 and 2, as talazoparib exhibits an affinity in the low nanomolar range for those ubiquitous proteins which is among others expressed in muscle tissue (see Fig. 13 for IC50). This might also explain the high bone (or skeletal muscle) uptake, since the radiotracer is highly stable in both mouse and human serum, however, liver metabolism might as well defluorinate [¹⁸F] talazoparib *in vivo* in mice as it was observed to a lower extent for [¹⁸F] PARPi.

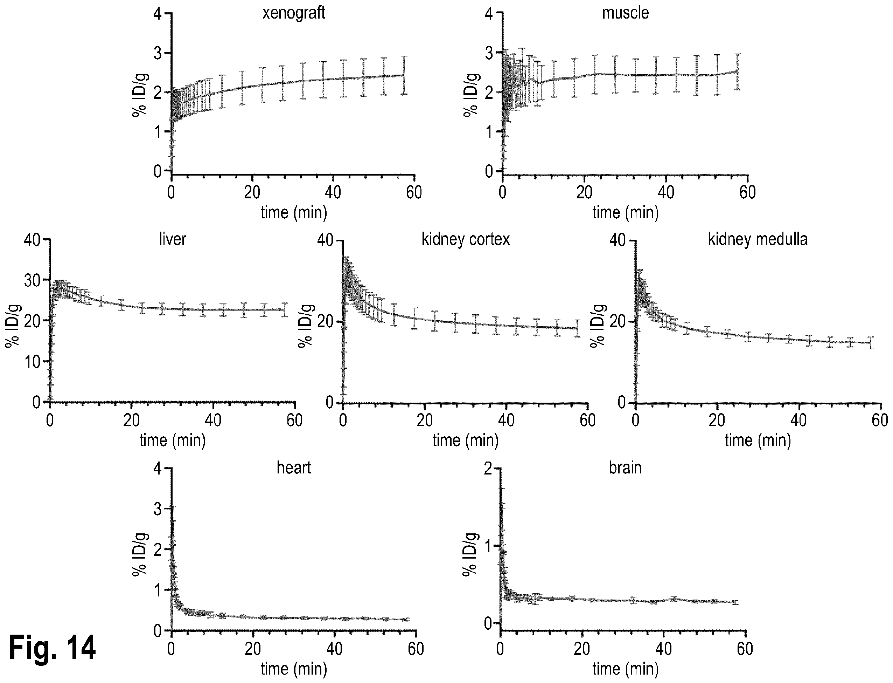

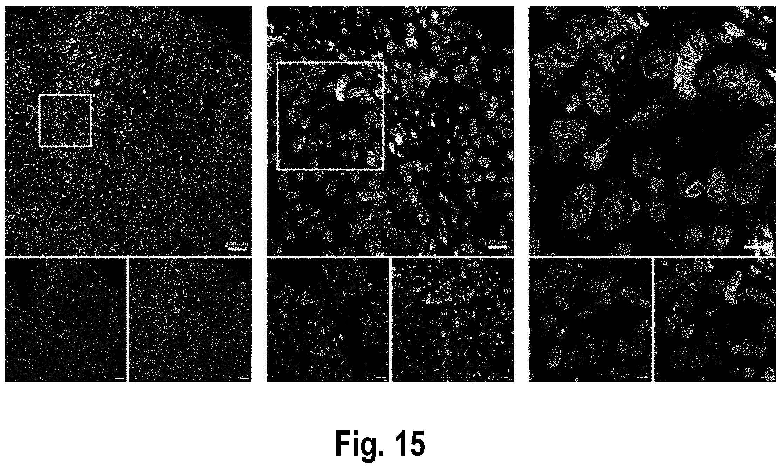

The liver-to-kidney ratio (LKR) gives first insights in the metabolism of [¹⁸F] talazoparib, with a mean LKR of 1.1 ± 0.09 indicating a balanced clearance by both renal and hepatobiliary pathways. A closer analysis of the pharmacokinetic profile by time-activity curve (TAC) analysis of several organs in the first hour after radiotracer administration verified this statement and shows similar uptake patterns and absolute values in liver and kidney (Fig. 14). As already expected, the radiotracer uptake in muscle and xenograft is comparably high during the first hour after radiotracer administration. The brain TAC shows an initial injection peak but the radiotracer is washed out rapidly and stays on a low level thereafter. With the blood TAC, fast blood clearance with a calculated blood half-life of 3.3 minutes was determined. PARP1 expression in the xenograft tissue was verified ex vivo by PARP1 immunofluorescence (Fig. 15).

### 3. Conclusions

The inventors proved general feasibility of [¹⁸F] talazoparib imaging and verified decent xenograft uptake, although overshadowed with high background uptake in muscle and bone. If referenced to blood, [¹⁸F] talazoparib outperformed [¹⁸F] PARPi in the same in vivo mouse model, warranting the exploration of [¹⁸F] talazoparib as an alternative PARP imaging agent with different indication.

The successful first synthesis of [¹⁸F] talazoparib is not only of great interest for nuclear medicine, but also proves the crucial role of copper-mediated radiofluorination for the development of novel radiotracers. This work lays the foundation for a deeper understanding of the pharmacokinetics of talazoparib and the exploitation of the different insights in its mechanistic for patient stratification.

As novel PARP radiotracer based on a second-generation PARP inhibitor, [¹⁸F] talazoparib gives the opportunity to visualize different aspects of PARP than the gold-standard radiotracers [¹⁸F]PARPi and [¹⁸F]FTT. Since it exhibits very low uptake in the brain tissue, [¹⁸F] talazoparib should be examined in glioblastoma models to determine its PARP binding in a setting in which the observed high muscle uptake plays only a minor role. Alternative cell models for evaluation of [¹⁸F] talazoparib could also include tankyrase-overexpressing cells, cell lines that express different PARP enzymes to assess binding besides PARP1/2, and tumor entities that reportedly respond well to talazoparib treatment in comparison to non-responding tumors.

The exact role of tankyrases in disease development and progression is still mainly unclear as they are involved in many essential biological processes like Wnt/β-catenin signaling, telomerase maintenance, regulation of insulin sensitivity, and mitosis regulation. Shedding light on tankyrases by in vivo PET imaging with [¹⁸F] talazoparib would aid future drug development as tankyrase inhibitors are currently investigated in their role as anti-cancer drugs.

If further studies verify the greater value of [¹⁸F] talazoparib for PARP imaging, clinical translation could be accelerated since the isotopically labeled molecule is chemically equal to the approved PARP inhibitor talazoparib. For GMP production of the radiotracer, the precursor should be separated in the two enantiomers before radiosynthesis, rendering the separation of the radiolabeled racemic mixture as described here redundant.

The inventors' study demonstrated feasibility of [¹⁸F] talazoparib-PET as novel tool for cancer and PARP imaging establishment of this crucial molecule.

## Claims

1. A compound of the formula 10 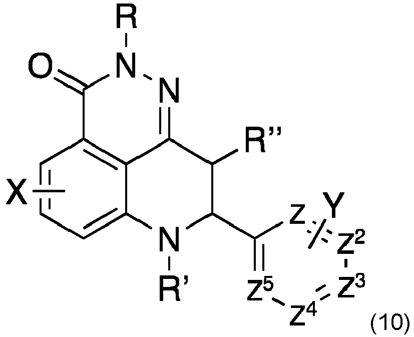 , wherein
R and R' are both H;
R" is a heteroaromatic or an aromatic residue, wherein R" has a molecular weight of 90 g/mol or less;
X and Y are independently selected from the group consisting of H, one or more leaving groups for [¹⁸F] fluorination, and a saturated or unsaturated alkyl residue of a molecular weight of 80 g/mol or less, provided that at least one leaving group for [¹⁸F] fluorination is present;
z and z²⁻⁵ are independently selected from the group consisting of C and N;
for use in the diagnosis of cancer.

2. The compound for use of claim 1, wherein said cancer is selected from the group consisting of: oral cancer, brain cancer, pancreatic cancer, liver cancer, breast cancer, and ovarian cancer.

3. The compound for use of claim 1 or 2, which is [¹⁸F] talazoparib.

4. The compound for use of any of claims 1-3, wherein said diagnosis is made by an imaging method.

5. The compound of claim 4, wherein said imaging method is positron emission tomography (PET).

6. A use of the compound of the formula 10 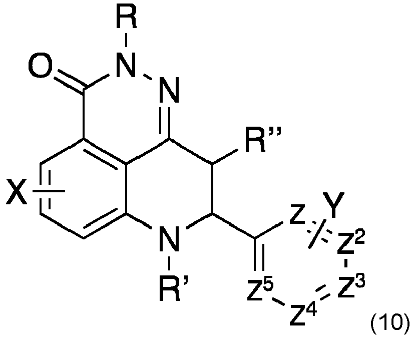 , wherein
R and R' are both H;
R" is a heteroaromatic or an aromatic residue, wherein R" has a molecular weight of 120 g/mol or less;
X and Y are independently selected from the group consisting of H, one or more leaving groups for [¹⁸F] fluorination, and a saturated or unsaturated alkyl residue of a molecular weight of 80 g/mol or less, provided that at least one leaving group for [¹⁸F] fluorination is present;
z and z²⁻⁵ are independently selected from the group consisting of C and N;
for visualizing a poly [ADP-ribose] polymerase (PARP) enzyme.

7. The use of claim 6, wherein said PARP enzyme is tankyrase, preferably tankyrase 1 and/ or tankyrase 2.

8. The use of claim 6 or 7, wherein said compound of formula 10 is [¹⁸F] talazoparib.

9. The use of any of claims 6-8, wherein said visualizing is made by an imaging method.

10. The use of claim 9, wherein said imaging method is positron emission tomography (PET).

11. A pharmaceutical composition comprising the compound of any of claims 1-5 and a pharmaceutically acceptable carrier.

12. The pharmaceutical composition of claim 11, which is a radiopharmaceutical.

13. A method for the diagnosis of cancer in a living being, comprising the step of introducing the compound of any of claims 1-5 or the pharmaceutical composition of claim 11 or 12 into said living being, and a step of visualizing said cancer by an imaging method, preferably by positron emission tomography (PET).

14. A method of visualizing a poly [ADP-ribose] polymerase (PARP) enzyme in biological material, comprising the step of introducing the compound of any of claims 1-5 into said biological material, and a step of visualizing said PARP enzyme by an imaging method, preferably by positron emission tomography (PET).

15. The method of claim 14, **characterized in that** said PARP enzyme is tankyrase, preferably tankyrase 1 and/ or tankyrase 2.
